# EUROPÄISCHE PATENTSCHRIFT

(11) **EP 2 614 848 B1**
(45) Veröffentlichungstag und Bekanntmachung des Hinweises auf die Patenterteilung: **01.07.2020**
(21) Anmeldenummer: 12151105.9
(22) Anmeldetag: 13.01.2012
(51) Int. Cl.: A61M 15/00, A61K 9/48, A61K 9/00, A61J 3/07, B65D 47/28

(54) **Inhalator und Kapsel für einen Inhalator**
Inhaler and capsule for an inhaler
Inhalateur et capsule pour inhalateur

(43) Veröffentlichungstag der Anmeldung: 17.07.2013
(73) Patentinhaber: Boehringer Ingelheim International GmbH, 55216 Ingelheim am Rhein (DE)
(72) Erfinder: Kladders, Heinrich, 55216 INGELHEIM AM RHEIN (DE)
(74) Vertreter: Simon, Elke Anna Maria

(56) Entgegenhaltungen:
- WO-A1-01/72605
- WO-A2-2006/074982
- DE-A1-102004 040 928
- FR-A- 1 215 560
- FR-A2- 2 032 436
- US-A- 4 076 848
- US-A1- 2004 131 668
- US-A1- 2004 173 211

## Beschreibung

Die vorliegende Erfindung betrifft eine Kapsel zur Aufnahme von medizinischen Formulierungen zum Einsatz in einem Inhalator und einen zugehörigen Inhalator. Insbesondere bezieht sich die Erfindung auf Kapseln, die mit einer pulverförmigen pharmazeutischen Zubereitung gefüllt sind und auf Inhalatoren, mit denen eine pulverförmige pharmazeutische Zubereitung zur Inhalation bereit gestellt werden soll, wobei sich das Pulver in einer Kapsel befindet und zur Inhalation mittels mindestens eines Lochs in der Kapselwand aus der Kapsel austritt.

Aus dem Stand der Technik sind Kapseln bekannt, die in bestimmten medizinischen Vorrichtungen wie Pulverinhalatoren zum Einsatz kommen. Die äußere Form von in solchen Inhalatoren verwendeten Kapseln ist häufig (wie auch in der vorliegenden Schrift) die eines geschlossenen Zylinders mit halbkugelförmigen Enden, wobei die Länge des Zylinders größer ist als sein Durchmesser. Solche Kapseln bestehen üblicherweise aus zwei becherartigen Teilen - einem Kapselkörper und einer Kapselkappe -, die teleskopartig ineinander gesteckt sind. Verschiedene Materialien solcher Kapseln sind bekannt. Viele in der Medizin verwendete Kapseln bestehen aus Gelatine oder Hartgelatine.
Die WO2000/07572 offenbart Kunststoffkapseln zur Verwendung in Pulverinhalatoren. Die Kapseln bestehen aus Kapselkörper und Kapselkappe, die so miteinander verbunden werden können, dass ein stabiler, abgeschlossener Hohlraum von definiertem Volumen gebildet wird. Die Kapsel kann Rastelemente aufweisen, welche die Kapselkappe fest mit dem Kapselkörper verbinden. Ein Beispiel für solche Rastelemente sind punktförmige Erhebungen im Innenmantel der Kapselkappe, die in etwas größere punktförmige Vertiefungen am Außenmantel des Kapselkörpers einrasten. Kapselkappe und Kapselkörper bestehen beide aus dem gleichen nicht-wasserlöslichen, hydrophoben Kunststoff - vorzugsweise Polyethylen. Die WO2006/074982 A2 offenbart ein Verschlusskonzept für Kapselkappe und Kapselkörper, durch dass es möglich ist, die beiden Teile für den Transport der Kapsel zur Befüllanlage provisorisch über einen Vorverschluss zu verbinden, der im Gegensatz zum Hauptverschluss zerstörungsfrei geöffnet werden kann. Die Verschlüsse werden im Innenmantel der Kapselkappe durch ringförmig verlaufende oder segmentartige Erhebungen und dazu passende peripher den Außenmantel des Kapselkörpers ringförmig umlaufende Vertiefungen gebildet.

Aus anderen technischen Gebieten sind darüber hinaus kleinformatige Aufbewahrungsgefäße für Pulver bekannt. So offenbart die FR 1215560 A ein zum Mitführen durch den Anwender geeignetes Aufbewahrungsgefäß für pharmazeutische Pulver, insbesondere Haftpulver für Zahnprothesen, umfassend ein Röhrchen, das an seinem offenen Ende durch eine Kappe verschlossen ist. Hierbei wird die Kappe alleine oder zusammen mit der Röhre mit einem oder mehreren Löchern zum Ausbringen des Pulvers versehen, und die Löcher können durch eine schiebende oder rotierende Bewegung der Kappe geschlossen werden.
Die US 4076848 offenbart ein bis eineinhalb Inch lange zylindrische Kapseln zur Aufbewahrung von getrockneten, pulverisierten Früchten oder Gemüse, wobei durch Verschieben oder Verdrehen von Kapselhälften gegeneinander Öffnungen freigelegt werden und der Kapselinhalt durch diese Öffnungen ins Essen geschüttet werden kann.

Aus dem Stand der Technik sind verschiedene Pulverinhalatoren bekannt, bei denen sich das Pulver vor der Inhalation in Kapseln befindet. In diesen Geräten werden die Kapseln in der Regel in irgendeiner Weise geöffnet, um das Pulver für die Zerstäubung zur Verfügung zu stellen: In manchen Geräten werden die Kapseln beispielsweise mit Messerklingen aufgeschnitten, in anderen wird ihr Inneres über Hohlnadeln an Luftwege im Inhalator angeschlossen. Bei einer Gruppe von Inhalatoren, die insbesondere den Hintergrund zu der hier vorliegenden Erfindung bilden, werden mit Hilfe von Nadelwerkzeugen Löcher in die Kapseln gestochen.
Die WO2004/082750 A1 zeigt ein Beispiel für einen solchen Inhalator, in dem eine Kapsel an ihren beiden Enden durch zwei entgegengesetzte Nadeln angestochen. Beim Inhalationsvorgang rotiert die Kapsel um ihre Querachse, wobei sie durch tangential einströmende Luft angetrieben wird. Partikel, die durch die Rotation der Kapsel aus ihrem Innern gefördert werden, bewegen sich dann durch den Luftstrom zum Mundstück.
Die US 5896855 zeigt einen Inhalator, in denen mehrere Kapseln in einem drehbaren Magazin bevorratet sind und die durch einen - wahlweise motorgesteuerten - Mechanismus einer Wirbelkammer zugeführt werden, wo das Pulver ebenfalls durch ein Rotieren der Kapsel um ihre Querachse aus Löchern an den Kapselenden ausgebracht wird. Im Magazin werden die Kapseln von Nadeln oder Stöpseln an beiden Enden festgehalten. Hierbei werden die Kapseln entweder vor Einsetzen in das Magazin an ihren Pol-Enden angestochen und diese Löcher werden durch die Stöpsel im Magazin bis zur Zuführung der jeweiligen Kapsel in die Wirbelkammer verschlossen; oder die Kapseln werden durch eben diese Nadeln beim Einsetzen der Kapseln ins Magazin angestochen und die Anstechnadeln verbleiben bis zur Zuführung der jeweiligen Kapsel in die Wirbelkammer abdichtend in den Löchern.

Die WO04/052435 A1 und die US 2004/173211 A1 zeigen verschiedene Kapsel-basierte Pulverinhalatoren, in denen die Zerstäubung unter der Nutzung des so genannten Bernoulli-Effekts stattfindet. Hierbei befindet sich eine die Pulverformulierung enthaltende Kapsel in einer Kammer, die mit einer Vorrichtung zur seitlichen Öffnung der Kapsel vorgesehen ist. Die Kapsel kann sich im Wesentlichen nur in Längsrichtung in der Kammer bewegen und wird durch eine Luftströmung parallel zur Längsachse der Kammer bewegt. Ein gezeigter Inhalator weist ein Mundstück auf, das ähnlich wie eine Kappe ausgebildet ist und auf ein Unterteil aufgesetzt wird, das die Kapselkammer beinhaltet. Am Gehäuseunterteil ist eine Schneidevorrichtung zum Öffnen der Kapseln vorgesehen. Zum Austausch der verbrauchten Kapseln gegen frische wird das Mundstück hochgeklappt oder eine Steckverbindung gelöst, die sich zwischen Mundstück und Gehäuseunterteil bzw. zwischen Mundstück und einer ins Gehäuseunterteil eingesetzten und mit der Kapselkammer verbundenen Platte befindet. Ein anderer gezeigter Inhalator weist ein drehbar gelagertes, austauschbares oder nachfüllbares Revolvermagazin mit mehreren, je mit einer Kapsel bestückten Kammern auf.

Solche den Bernoulli-Effekt nutzenden Pulverinhalatoren bilden den Ausgangspunkt für die hier vorgestellte Erfindung und die im Folgenden beschriebene Funktionsweise gilt auch für die Inhalatoren, die Gegenstand der vorliegenden Erfindungen sind.
In den hier betrachteten Inhalatoren ist der auszubringende Wirkstoff in einer im Wesentlichen zylindrischen Kapsel gelagert und diese Kapsel wird in die Inhalationskammer eines Inhalators eingesetzt. Die Kapselkammer ist dabei an die Größe der Kapsel insofern angepasst, dass sie ebenfalls im Wesentlichen zylindrisch ausgebildet ist, wobei ihre Länge und ihr Durchmesser jeweils etwas größer als die entsprechende Maße der Kapsel sind. Dadurch hat eine in die Kapselkammer eingesetzte Kapsel genug Spielraum, um sowohl in axialer als auch in radialer Richtung Vibrationsbewegungen ausführen zu können, dabei aber im wesentlichen entlang der Kammerachse ausgerichtet bleibt. Die Kapselkammer weist im Bereich eines der beiden Enden einen Lufteinlass und im Bereich des anderen Endes eine Luftauslassöffnung auf. Der Luftauslass ist an einen Inhalationskanal angeschlossen, der zum Mundstück des Inhalators führt. In der Regel sind Kapselkammer, Luftauslass, Inhalationskanal und Öffnung im Mundstück entlang einer gemeinsamen Achse angeordnet. Zum Ausbringen des Inhalts der Kapsel wird die Kapsel zunächst an üblicherweise zwei Stellen längsseits am Mantel geöffnet. In der Regel befinden sich die Öffnungen in der Nähe der beiden längsseitigen Enden der Kapsel. Wird nun in der Kapselkammer ein Luftstrom vom Lufteinlass zum Luftauslass erzeugt, führt dieser entlang der Längsachse der Kapsel und bewirkt dabei zweierlei: Zum einen vibriert die Kapsel, wobei ihre Vorzugsbewegungsrichtung bedingt durch den Luftstrom entlang der Längsachse verläuft. Zum anderen erzeugt die an den beiden Kapselöffnungen entlang strömende Luft gegenüber dem Kapselinneren einen Unterdruck, so dass das in der Kapsel befindliche Pulver vom Luftstrom mitgerissen und dabei zerstäubt wird.

Der vorliegenden Erfindung liegt die Aufgabe zugrunde, eine gegenüber dem Stand der Technik verbesserte Kapsel für die Verwendung in Inhalatoren und einen verbesserten Inhalator anzugeben. Insbesondere soll ein System aus Kapsel und Inhalator angegeben werden, bei dem die Reproduzierbarkeit der Zerstäubung, insbesondere die Pulverausbringung aus der Kapsel verbessert wird. Vorzugsweise soll ein System angegeben werden, bei dem Unregelmäßigkeiten beim Öffnen der Kapseln reduziert oder minimiert werden.

Die genannte Aufgabe wird erfindungsgemäß gelöst durch eine Kapsel für die Verwendung als Reservoir für eine pharmazeutische Zubereitung bzw. medizinische Formulierung in einem Inhalator, wobei die Kapsel zwei einseitig offene Kapselelemente, nämlich einen Kapselkörper und eine Kapselkappe, umfasst, die zur Bildung eines Hohlraums teleskopartig über ihre Öffnungen ineinander gesteckt werden können. Kapselkörper und Kapselkappe sind dabei dadurch gekennzeichnet, dass beide zusätzlich zur einseitigen Öffnung je zwei vorgefertigte Löcher aufweisen.

Des Weiteren wird die erfindungsgemäße Aufgabe gelöst durch einen Inhalator, der eine Kapselkammer mit einem Lufteinlass und einem in Richtung eines Mundstücks führenden Luftauslass aufweist, wobei eine Kapsel in die Kapselkammer eingesetzt oder aus einem Vorratsmagazin in die Kapselkammer eingeführt werden kann und der Inhalator einen Schieber aufweist, bei dessen Betätigung Teile der Kapsel so gegeneinander verschoben werden können, dass an der Kapsel vorgefertigte Löcher freigegeben werden.

Des Weiteren wird die Aufgabe erfindungsgemäß gelöst durch ein System, das aus der genannten Kapsel und dem genannten Inhalator gebildet wird.

Vorteilhafte Weiterbildungen werden im Folgenden und im Detail anhand der Figuren beschrieben.

Ein Merkmal der vorliegenden Erfindung ist, dass die zusammengesetzte Kapsel in zwei verschiedenen Zuständen vorliegen kann: Im ersten Zustand ist das vorgefertigte Loch geschlossen, in der zweiten liegt es frei. In dem Inhalator, in dem diese Kapsel eingesetzt wird, wird die Kapsel von ihren ersten in den zweiten Zustand überführt wird, in dem dann die pharmazeutische Zubereitung über das vorgefertigte Loch aus der Kapsel austreten kann.

Ein weiteres Merkmal der vorliegenden Erfindung ist, dass beide Kapselelemente vorgefertigte Löcher und beim teleskopartigen Ineinanderstecken zwei Einsteckstellungen zueinander aufweisen (Diese beiden Einsteckstellungen entsprechen in dieser Ausführung dem so genannten ersten und zweiten Zustand der Kapsel): Eine erste Einsteckstellung, in welcher beide Elemente so ineinander gesteckt sind, dass die vorgefertigten Löcher verdeckt sind und der Hohlraum der Kapsel insgesamt geschlossen ist, und eine zweite Einsteckstellung, in der sich die vorgefertigten Löcher in Kapselkörper und Kapselkappe so überdecken, dass die gesamte Kapsel an der Stelle der Überdeckung beider Löcher ein Loch aufweist.
Vorzugsweise sind beide Kapselelemente becherartig geformt: Der von ihnen gebildete, einseitig offene Hohlraum wird seitlich durch einen umlaufenden Kapselmantel und gegenüber der offenen Seite durch ein geschlossenes Ende begrenzt. Vorzugsweise bildet der Kapselmantel eine zylindrische oder elliptisch umlaufende Wand, so dass in der zusammengeschobenen Kapsel innen keine Ecken gebildet werden, in denen sich pulverförmige pharmazeutische Zubereitung ansammeln und dadurch bei einer späteren Ausbringung des Pulvers aus der Kapsel zurück bleiben könnte. Aus gleichem Grund haben die Unterseiten von Kapselkörper und Kapselkappe und somit beide Enden der beim Ineinanderstecken resultierenden Kapsel eine konvexe, insbesondere im Wesentlichen halbkugelartige oder ellipsoidale Form.
Die vorgefertigten Löcher in Kapselkappe und Kapselkörper befinden sich in ihrem jeweiligen Mantelbereich, so dass beim Ineinanderstecken jeweils der Mantel des anderen Kapselelements das jeweilige Loch abdeckt, bis die Einsteckstellung erreicht ist, in der beide Löcher zur Überdeckung gebracht werden.
Darunter, dass ein Loch "vorgefertigt" ist, ist zu verstehen, dass das Loch in dem jeweiligen Kapselelement bereits im werksseitigen Herstellprozess der Kapseln entsteht. Das Loch im jeweiligen Kapselelement ist hierbei bereits vorhanden, bevor die einzelne Kapsel fertig zusammengesetzt werden. Insbesondere sind die Löcher in den Kapselelementen bereits vorhanden, bevor die Kapsel als ganzes in einen Inhalator eingesetzt wird.

Bevorzugt sind Kapselkappe und Kapselkörper auf jeweils den Seiten, die im ineinandergesteckten Zustand aneinander liegen, im Kapselmantelbereich strukturiert. Diese Strukturierung ist vorzugsweise so ausgelegt, dass sie unterschiedliche Funktionen erfüllt. Zum einen weisen die Strukturen von Kapselkappe und Kapselkörper wechselseitige Rastelemente auf. Bevorzugt bewirkt diese Struktur beim Ineinanderstecken der Kapselelemente, dass die Kapselkappe und der Kapselkörper in Relation zu einander in mindestens zwei Stellungen, insbesondere den genannten Einsteckstellungen, verrasten. Durch eine Verrastung in der ersten Einsteckstellung wird sichergestellt, dass zum einen die Löcher in den Kapseln nicht zufällig z.B. durch Erschütterungen beim Transport vorzeitig freigelegt werden und ein definierter Vorgang wie das Zusammenschieben beider Elemente unter Ausübung eines definierten Drucks notwendig ist, um die Löcher freizulegen. Des Weiteren sind die miteinander wechselwirkenden Rastelemente von Kapselkappe und Kapselkörper vorzugsweise so gestaltet, dass sich die beiden Kapselelemente nach dem Ineinanderstecken bis zur ersten Einsteckstellung nicht mehr zerstörungsfrei von einander trennen lassen. Dadurch wird verhindert, dass sich die Kapsel zufällig öffnet.
Vorzugsweise sind im Kontaktbereich zwischen Kapselkappe und Kapselkörper zusätzlich Strukturelemente vorhanden, die als Führung beim Ineinanderstecken der beiden Kapselelemente dienen. Diese Strukturelemente an den zueinander gerichteten Mantelbereichen von Kapselkappe und Kapselkörper bewirken, dass die beiden Kapselelemente nur in definierten Ausrichtungen ineinander gesteckt werden können. Die "definierte Ausrichtung" bezieht sich auf die Drehung der Kapselelemente um die Längsachse der Kapsel, es ist also eine azimutale Ausrichtung. Vorzugsweise haben diese Strukturelemente die Gestalt mindestens einer Nut in der äußeren / inneren Mantelfläche von Kapselkappe / Kapselkörper und respektive die Gestalt mindestens einer Führungsschiene in der inneren / äußeren Mantelfläche von Kapselkörper / Kapselkappe. Auf diese Weise wird sichergestellt, dass beim Ineinanderstecken der Kapselelemente die vorgefertigten Löcher in Kapselkappe und Kapselkörper sicher zur Überdeckung gebracht werden können. Bevorzugt werden hierbei geradlinige Führungen, insbesondere solche parallel zur Hauptachse der Kapsel. Varianten in denen die Strukturelemente die Bewegung der Kapselelemente beim Ineinanderstecken gekurvt führen sind ebenfalls möglich. Dies würde beispielsweise durch eine schraubenförmigen Führungsschiene bewirkt werden.

Ein weiteres - sowohl unabhängig als auch in Kombination mit den oben genannten Aspekten umsetzbares - Merkmal der vorliegenden Erfindung ist, dass die Kapsel statt eines zylindrischen Querschnitts einen elliptischen Querschnitt aufweist. Vorzugsweise handelt es sich bei der Ellipse um so eine, die nur leicht von der Form eines Kreises abweicht (Das Verhältnis von Längs-zu-Querachse der Ellipse sollte kleiner als 75%, vorzugsweise zwischen 90% und 85% sein). Ein solcher elliptischer Querschnitt erzwingt eine definierte azimutale Ausrichtung der beiden Kapselelemente beim Ineinanderstecken.

Ein weiteres Merkmal der vorliegenden Erfindung ist, dass das Loch im äußeren Kapselelement vorzugsweise als Langloch, elliptisch oder etwas größer als das zugehörige Loch im inneren Kapselement gestaltet ist. Bei Ausführungen des äußeren Lochs als Langloch oder als Ellipse ist vorzugsweise der schmale Durchmesser des Lochs mindestens so groß wie der Durchmesser des Lochs im inneren Kapselelement. Das äußere Kapselelement ist jenes, das von Kapselkörper und Kapselkappe im ineinandergesteckten Zustand im Bereich der aneinanderliegenden Kapselmäntel die Außenwand der Kapsel bildet. Durch diese Vergrößerung des äußeren Lochs gegenüber dem inneren werden die Toleranzen hinsichtlich der Genauigkeit des Ineinanderstecks der Kapselelemente aufgeweitet. Somit wird sichergestellt, dass auch bei unpräzisem Aufeineinanderschieben die volle Kapselöffnung zur Verfügung steht.

Ein weiteres Merkmal der vorliegenden Erfindung ist, dass eine mit einer pharmazeutischen Zubereitung gefüllte Kapsel, die eine Kapselkappe und/oder einen Kapselkörper mit vorgefertigten Löchern aufweist, in einem Inhalator verwendet wird, wobei die vorgefertigten Löcher zum Zeitpunkt des Einsetzens der Kapsel in eine Kapselkammer des Inhalators verdeckt sind und durch Betätigung eines Schiebers im Inhalator freigelegt werden.
Dies ermöglicht die Bereitstellung eines kapselbasierten, vorzugsweise den Bernoulli-Effekt zur Zerstäubung nutzenden, Inhalators, in dem keine Anstechvorrichtungen zur Lochung der Kapsel bereit gestellt werden müssen. Das Freilegen von vorgefertigten Löchern bietet gegenüber der Verwendung von Anstechvorrichtungen im Inhalator verschiedene Vorteile: Die vorgefertigten Löcher sind bedingt durch ihren Fertigungsprozess - z.B. KunststoffSpritzguss - in Größe und Form von Kapsel zu Kapsel sehr gut reproduzierbar während durch Anstechen erzeugte Löcher in Abhängigkeit von Kapselwerkstoff, -größe und Material so wie Anstechposition und Geometrie der Nadeln individuell variieren und/oder zu unregelmäßigen Lochgeometrien führen können. Des Weiteren ist beim Anstechen von Kapseln eine gewisse Rückformung der Kapseloberfläche im Bereich der Einstechstelle möglich. Die hier vorgestellten, vorgefertigten Löcher hingegen haben nach ihrer Freilegung eine stabile Form und keine Vorsprünge im Kapselmaterial. Solche Vorsprünge im Kapselmaterial bilden sich z.B. beim Eindrücken der Kapselwand durch eine Anstechvorrichtung und können möglicherweise zur Anlagerung von insbesondere pulverförmiger pharmazeutischer Zubereitung und somit bei Zerstäubung zu einer leicht verminderten Ausbringung der Zubereitung aus der Kapsel führen. Aufgrund der nun durch die Vorfertigung der Löcher möglichen hohen Präzisierung von der Größe und Form der Kapsellöcher sind sowohl die Menge als auch insbesondere die Verteilung des Pulvers reproduzierbarer, das aus Kapseln im Luftstrom in der Kapselkammer eines Inhalators ausgebracht wird. Die Zerstäubung der pharmazeutischen Zubereitung selbst wird dadurch reproduzierbarer, insbesondere im Vergleich zu Inhalatoren mit Kapselanstechvorrichtungen.

In weiteres Merkmal der vorliegenden Erfindung ist, dass der Inhalator zur Verwendung mit Kapseln mit vorgefertigten Löchern einen Schieber aufweist und dieser Schieber zum Freilegen der vorgefertigten Kapsellöcher eine Begrenzung des Innenraums der Kapselkammer, insbesondere einen Teil der Wand der Kapselkammer selbst bildet. Für das Öffnen der Kapsel müssen somit an der Kapselkammer keine zusätzlichen Öffnungen ausgebildet werden, die das Strömungsverhalten bei der Zerstäubung nachteilig beeinflussen können. Es kommt nicht zur Bildung von unerwünschter Seitenluft bzw. Falschluft neben den für die Zerstäubung benötigten Luftkanälen. Das System ist hinsichtlich des Strömungswiderstandes stabil und reproduzierbar.
Vergleichsweise müsste bei der Verwendung einer Anstechvorrichtung das zugehörige Anstechelement durch eine oder mehrere Öffnungen in die Kapselkammer eingeführt werden, was während der Inhalation zu zusätzlichen unerwünschten Luftströmungen durch diese Öffnungen führen kann. Solche Öffnungen zusätzlich zu Lufteinlass und Luftauslass der Kapselkammer werden beim erfindungsgemäßen Inhalator nicht benötigt, sofern sie nicht ihrerseits auf eine Verbesserung der Luftströmung in der Kapselkammer ausgerichtet sind. Die Verwendung eines Schiebers zum Freilegen von vorgefertigten Löchern hat im Vergleich zur Verwendung von Anstechelementen den weiteren Vorteil, das auch bei mehrfacher Anwendung keine Abnutzungserschienungen oder Materialablagerungen zu erwarten sind. Vergleichsweise können sich bei Inhalatoren mit Anstechvorrichtungen, die mehrmals zum Anstechen verwendet werden - z.B. bei Inhalatoren, in denen nach Benutzung die alte Kapsel in der Kapselkammer regelmäßig durch eine unbenutzte ersetzt wird -, neben einer Abnutzung der Schneidkanten oder Spitzen insbesondere bei der Verwendung von teilweise klebrigen, pulverförmigen pharmazeutischen Zubereitungen auch Materialablagerung an den Anstechspitzen bilden. Diese Ablagerungen können im schlimmsten Fall einerseits zu geringen Dosisveränderungen bei der Ausbringung aus dem Inhalator führen - sei es durch Verbleib solcher Ablagerungen an der neuen Kapsel oder durch Bildung dieser Ablagerungen aus dem Inhalt einer neuangestochenen Kapsel. Zum anderen können diese Ablagerungen an den Anstechspitzen genauso wie Verschleißerscheinungen an den Spitzen die Geometrie der Löcher in den Kapseln beeinflussen und Unregelmäßigkeiten in Größe und Form der Löcher hervorrufen, wodurch wiederum die Reproduzierbarkeit der Ausbringung der pharmazeutischen Zubereitung aus den Kapsel beeinträchtig wird. Des Weiteren kann es, bedingt durch den für den Zerstäubungsprozess notwendigen Bewegungsspielraum der Kapsel in der Kapselkammer, zu Schwankungen bei der Position der Kapsel zu den Anstechspitzen kommen, wenn die Kapsel in der Kapselkammer angestochen wird. Dies führt beim Anstich mehrerer Kapseln zu Schwankungen bezüglich der exakten Position der Löcher in der Kapsel.
Durch die Verwendung eines Schieber-Mechanismus zur Freilegung von vorgefertigten Löchern an Kapseln werden alle aus der Verwendung von Anstechvorrichtungen resultierenden Schwankungen in der Form, Größe und Position der Löcher und die sich daraus ergebenden Schwankungen in der Ausbringung der pharmazeutischen Zubereitung vermieden. Die Zerstäubung der pharmazeutischen Zubereitung ist nun unabhängig von dem Mechanismus zum Öffnen der Löcher in der Kapsel. Dies bedeutet auch, dass bei Verwendung von brüchigem Kapselmaterial das Risiko einer Splitterung, wie sie insbesondere bei Anstechvorgängen auftreten könnte, minimiert ist. Auf diese Weise erweitert sich die Gruppe möglicher Kapselmaterialien für die Verwendung in Inhalatoren.

Die Verwendung eines Schiebemechanismus zum Freilegen vorgefertigter Löcher hat des Weiteren den Vorteil, dass der gleiche Mechanismus für die Freilegung unterschiedlichster Lochanordnungen an Kapseln benutzt werden kann. Z.B. können sich bei zylindrischen Anordnungen von Kapsel, Kapselkammer und Schieber mehrere Löcher an der Kapsel an - bezogen auf ihren Umfang - unterschiedlichen Stellen befinden. Größe, Form, Position, Anzahl der Löcher in einer Kapsel sind bei diesem Konzept sehr variabel - insbesondere bei einer Fertigung der Kapselteile aus Spritzgussprozessen. An dieser Stelle können die Ergebnisse von auf die Zerstäubung von bestimmten pharmazeutischen Zubereitungen bzw. bestimmten Pulvern abgestimmten Strömungssimulationen benutzt werden, um optimale Lochstrukturen für Kapseln für diese bestimmte Zubereitung bzw. dieses bestimmte Pulver zu verwenden.

Ein Merkmal eines weiteren Konzepts (nicht zur Erfindung gehörend)ist, dass in einer alternativen Ausführungsform die in der Kapsel vorgefertigten Löcher durch ein ebenfalls zur Kapsel gehörendes, zusätzliches Bauteil verschlossen sind. Bei im Wesentlichen zylindrischen Kapseln mit vorgefertigten Löchern im Kapselmantel hat dieses zusätzliche Bauteil eine ringförmige Struktur. Dieser Ring wird von außen so entlang der Längsachse über die Kapsel geschoben, dass er die Löcher dicht verschließt.

In Abhängigkeit vom Abdichtungskonzept der vorgefertigten Löcher in der Kapsel wird der Schieber ausgelegt, mit dessen Hilfe die Löcher freigelegt werden. Bei Kapseln, deren Löcher bei Einsetzen in die Kapselkammer mit einem Ring verschlossen sind, hat der Schieber vorzugsweise die Gestalt eines Hohlzylinders oder mehrerer seitlich an der Kapsel vorbeigreifenden Stempel, die den Ring von der Kapsel schieben und somit die Löcher freilegen. Auch hierbei bildet der Schieber (14) insbesondere eine Begrenzung des Innenraums der Kapselkammer (13).
Bei Kapseln, deren Löcher durch eine relative Verschiebung von Kapselkörper und Kapselkappe ineinander freigelegt werden, wirkt der Schieber auf ein Ende der Kapsel ein. Die wirksame Fläche des Schiebers ist hierbei flach oder vorzugsweise konkav gekrümmt, so dass sie flächig an dem Ende der Kapsel anliegt und die Kraft nicht nur in einem Punkt (dem Pol oder Scheitelpunkt des Kapselendes) übertragen wird. Der Schieber zum Zusammenschieben von Kapselelementen kann Teil der Kapselkammer und/oder Teil des Zuführmechanismus der Kapsel aus einem Vorratsmagazin sein, falls ein Inhalator mit der Bevorratung mehrerer Kapseln betrachtet wird. Insbesondere ist der Schieber (14) so ausgebildet, dass er eine Begrenzung des Innenraums der Kapselkammer (13) bildet.
Bei solch einem Inhalator mit Vorratsmagazin kann der Kapseltransport zur Kapselkammer beispielsweise aus einem Vorratsschacht erfolgen, bei dem die Kapseln quer durch eine sich über eine Rampe verjüngende Öffnung geschoben werden und diese verjüngende Öffnung das weitere Ineinanderschieben der Kapselelementen erzwingt. In einer solchen Ausführungsform wirkt der Schieber gegebenenfalls nur indirekt auf die Kapsel ein - nämlich als Druckstempel oben am Vorratsschacht.

In einer anderen Ausführungsform mit Vorratsmagazin werden die Kapseln aus einer Vorlageposition mit dem Schieber in die Kapsel hinein gedrückt. Mit dem Hineindrücken in die Kapselkammer erfolgt das zur Öffnung der Löcher notwendige weitere Ineinanderschieben von Kapselkappe und Kapselkörper.
Aufgrund der geringen Komplexität eines Schiebersystems und der damit verbundenen geringen Fertigungskosten, eignet sich ein solches System aus Kapsel mit vorgefertigten, vorerst verdeckten Löchern und in eine Kapselkammer intergriertem Schieber auch für die Fertigung von Wegwerf-Inhaltoren. Insbesondere bei Anwendungen, bei denen klebrige pulverförmige pharmazeutischen Zubereitungen zur Inhaltion bereit gestellt werden sollen, bilden sich schnell Ablagerungen in Kapselkammer, Luftauslass und Mundstück, so dass ein häufiger Austausch eines solchen Systems gewünscht ist, wie er durch ein Einweg-Produkt gegeben ist.

Die einzelnen Merkmale der vorliegenden Erfindung können unabhängig voneinander genutzt oder miteinander kombiniert werden.
Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnungen. Die Zeichnungen zeigen in:
Fig. 1 eine schematische Darstellung zum Funktionsprinzip der erfindungsgemäßen Kapsel in unterschiedlichen Zuständen: a) Kapselkappe und Kapselkörper vor dem Ineinanderstecken, b) Kapselkappe und Kapselkörper in einer ersten Einsteckstellung und c) Kapselkappe und Kapselkörper in einer zweiten Einsteckstellung;
Fig. 2 eine schematische Darstellung zum Funktionsprinzip der erfindungsgemäßen Kapsel anhand eines zweiten Referenzbeispiels in unterschiedlichen Zuständen: a) Kapselkappe und Kapselkörper vor dem Ineinanderstecken und b) Kapselkappe und Kapselkörper in einer zweiten Einsteckstellung;
Fig. 3 eine schematische Darstellung zum Funktionsprinzip der erfindungsgemäßen Kapsel anhand eines dritten Referenzbeispiels, wobei Kapselkappe und Kapselkörper vor dem Ineinanderstecken gezeigt werden;
Fig. 4 eine schematischen Querschnitt einer möglichen Ausführungsform der erfindungsgemäßen Kapsel, wobei in der ersten Bildhälfte Kapselkappe und Kapselkörper vor dem Ineinanderstecken und in der zweiten Bildhälfte Kapselkappe und Kapselkörper nach dem Ineinanderstecken gezeigt werden.
Fig. 5 eine schematischen Querschnitt einer weiteren möglichen Ausführungsform der erfindungsgemäßen Kapsel, wobei in der ersten Bildhälfte Kapselkappe und Kapselkörper vor dem Ineinanderstecken und in der zweiten Bildhälfte Kapselkappe und Kapselkörper nach dem Ineinanderstecken gezeigt werden.
Fig. 6 eine schematische Darstellung zur Funktionsweise eines Inhalators mit Vorratsmagazin zur Verwendung mit erfindungsgemäßer Kapseln.
Fig. 7 eine schematische Darstellung zur Funktionsweise einer zweiten Ausführungsform eines Inhalators mit Vorratsmagazin zur Verwendung mit erfindungsgemäßer Kapseln.
Fig. 8 eine schematische Darstellung zur Funktionsweise eines einmal verwendbaren Inhalators zur Verwendung mit einer erfindungsgemäßen Kapsel.
Fig. 9 eine schematische Darstellung zur Funktionsweise einer zweiten Ausführungsform eines einmal verwendbaren Inhalators zur Verwendung mit einer erfindungsgemäßen Kapsel.
Fig. 10 eine schematische Darstellung zur Funktionsweise einer dritten Ausführungsform eines einmal verwendbaren Inhalators zur Verwendung mit einer erfindungsgemäßen Kapsel.
Fig. 11 eine schematische Darstellung einer Ausführungsform einer alternativen (hier nicht erfindungsgemäßen) Kapsel in unterschiedlichen Zuständen: Fig. 11a zeigt Kapselkappe, Kapselkörper und Ring vor dem Ineinanderstecken, Fig. 11b zeigt Kapselkappe getrennt von den zusammengeschobenen Bauteilen Ring und Kapselkörper, Fig. 11c zeigt Kapselkappe und Kapselkörper in einer zweiten Einsteckstellung.
Fig. 12 eine schematische Darstellung zur Funktionsweise einer Ausführungsform eines einmal verwendbaren Inhalators zur Verwendung mit einer alternativen (nicht erfindungsgemäßen) Kapsel.

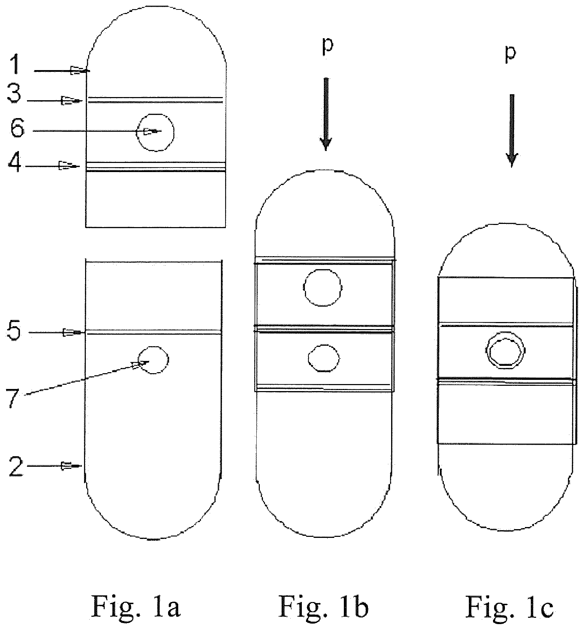

Figur 1 zeigt in schematischer Darstellung eine Kapsel erfindungsgemäß bestehend aus Kapselkappe (1) und Kapselkörper (2), die beide eine becherartige Form haben und teleskopartig über ihre Öffnungen ineinander gesteckt werden können. Diese und alle nachfolgenden Darstellungen sind als Skizzen zu verstehen, in denen Wandstärken und ähnliche Details nicht in vollem Umfang dargestellt bzw. teilweise nicht unbedingt maßstabsgerecht zueinander abgebildet sind.
Die in dieser und den folgenden Figuren dargestellten Kapseln werden mit einer pulverförmigen Arzneimittelzubereitung gefüllt. Bevorzugt haben die Kapselkappe und der Kapselkörper die Form eines auf einer Seite offenen Zylinders mit rundem Querschnitt und konvexer, nahezu halbkugelförmiger geschlossener anderer Seite. Kappe und Körper bestehen beide vorzugsweise aus Polypropylen oder Polyethylen, besonders bevorzugt aus high-density Polyethylen mit einer Dichte zwischen 950 und 1000 kg/m³. Die Kapselgrößen sind an die jeweiligen Inhalatoren bzw. die Dimensionen der darin enthaltenen Kapselkammern angepasst, in denen sie eingesetzt werden sollen. Typische Längen der zusammengefügten Kapseln sind beispielsweise 9 mm bis 22 mm bei äußeren Durchmessern von 4 mm bis 10 mm. Als Beispiele für die Kapseldimensionen gibt die WO2006/074982 A2 auf Seite 6 Zeile 6 bis 27 die folgende Wertebereiche an, wobei zusätzlich der Hinweis gegeben ist, dass die Kapselgrößen an die jeweiligen Inhalatoren angepasst sind:
- Länge der Kapselkörper: von ca. 22 bis ca. 9 mm, bevorzugt: 22,2 ±0,46 mm; 20,22 ±0,46 mm; 20,98 ±0,46 mm; 18,4 ±0,46 mm; 16,61 ±0,46 mm; 15,27 ±0,46 mm; 13,59 ±0,46 mm; 13,1 ± 0,1 mm;_12,19 ±0,46 mm; 9,3 ±0,46 mm.
- Länge der Kapselkappe: von ca. 13 bis 6 mm, ca. bevorzugt: 12,95 ±0,46 mm; 11,74 ±0,46 mm; 11,99 ±0,46 mm; 10,72 ±0,46 mm; 9,78 ±0,46 mm; 8,94 ± 0,46 mm; 8,54 ± 0,1 mm;_8,08 ±0,46 mm; 7,21 ±0,46 mm; 6,2 ±0,46 mm.
- Äußerer Durchmesser der Kapselkörper: von ca. 10 bis ca. 4 mm, bevorzugt: 9,55 mm; 8,18 mm; 7,36 mm; 7,34 mm; 6,63 mm; 6,07 mm; 5,57 ± 0,06 mm; 5,05 mm; 4,68 mm.
- Äußerer Durchmesser der Kapselkappen: von ca. 10 bis ca. 4 mm, bevorzugt: 9,91 mm; 8,53 mm; 7,66 mm; 7,64 mm; 6,91 mm; 6,35 mm; 5,83 ± 0,06 mm; 5,32 mm; 4,91 mm.
- Gesamtlänge der geschlossenen Kapsel: von ca. 27 bis ca. 11 mm, bevorzugt: 26,1 ±0,3 mm; 23,3 ±0,3 mm; 24,2 ±0,3 mm; 21,7 ±0,3 mm; 19,4 ±0,3 mm; 18,0 ±0,3 mm; 15,9 ±0,3 mm; 14,3 ±0,3 mm; 11,1 ±0,3 mm.
- Kapselvolumina: von ca. 1,4 bis ca. 0,1 ml, bevorzugt: 1,37 ml; 0,95 ml; 0,78 ml; 0,50 ml; 0,37 ml; 0,30 ml; 0,24 ml; 0,21 ml; 0,13 ml.
- Gewicht der Kapseln: von ca. 170 mg bis ca. 20 mg, bevorzugt zwischen 80 und 125 mg, bevorzugte Einzelwerte: 163 mg; 118 mg; 110 mg; 105 mg, 100 mg, 96 mg; 76 mg; 61 mg; 48 mg; 38 mg; 28 mg.

Bezüglich der Materialauslegung der Kapsel, für die neben dem hier bevorzugten Polyethylen auch alle pharmazeutisch akzeptablen Kunststoffe in Frage kommen, offenbart die WO2006/074982 A2 auf Seite 5 Zeile 6 bis 31 Folgendes:
"Als Kunststoffmaterial für die Kapsel kommen alle pharmazeutisch akzeptablen Kunststoffe in Frage, die spritz- oder blasformtechnisch sowie tiefziehtechnisch durch Thermoformen verarbeitet werden können und/oder Kunststoffe für deren Verarbeitung zur Kapselkappe oder zum Kapselkörper kein Formtrennmittel notwendig ist, das ein Anhaften der Inhaltsstoffe an die Kapselwand bewirken kann. Der Kunststoff sollte auch keine ausgeprägte Adhäsion für pharmazeutisch-chemische Stoffe, insbesondere für Partikel mit lungengängiger Größe aufweisen. Die bevorzugte Shorehärte D der Materialien liegt zwischen 10 und 85, bevorzugt zwischen 55 und 75, besonders bevorzugt 60 bis 70. Das Material sollte auch so sein, dass eine Kunststoffkapsel entlang der Längsache einer Kraft bis zu 20 N standhält. Außerdem sollte die Wand der Kapsel eine Durchlässigkeit für Wasserdampf von weniger als 1,3 x 10⁻¹⁴ kg/(m² s Pa), bevorzugt von 1,5 x 10⁻¹⁶ bis 2 x 10⁻¹⁶ kg/(m² s Pa) aufweisen. Die Schmelzeviskosität MFR (melt flow rate) liegt bevorzugt zwischen 40 und 65 g/10 Minuten, bevorzugt bei 45 - 59 g/10 Minuten und besonders bevorzugt bei 52 g/10 Minuten.
In bevorzugten Ausführungen ist der Kunststoff Polyethylen, insbesondere Polyethylen mit einer Dichte zwischen 900 und 1000 kg/m³, bevorzugt zwischen 960 und 970 kg/m³ (high-density Polyethylen). Daneben sind auch Polycarbonat, Polyester, Polypropylen, Polyethylenterephthalat, Polyutethan geeignet. Bevorzugt ist Poylethylen. Außerdem können geschäumte Kunststoffe verwendet werden, beispielsweise solche, wie sie z.B. unter dem Markennamen Hydrocerol® bekannt sind. Bei diesen Materialien handelt es sich um Kunststoffe, die während des Verarbeitungsvorgangs (Spritzguss) durch zugesetzte oder freigesetzte Schäumungsmittel aufgeschäumt werden. Als Schäumungsmittel können beispielsweise verwendet werden Azadicarbonamid (ADC), 4,4'-Oxybis(benzolsulfonyl-hydrazid) (BSH), 5-Phenyltetrazol (5-PT), p-Tuluylsulfonylsemicarbazid (TSS), p-Tuluylsulfonylhydrazid (TSH), verschiedene Citrate, Zitronensäure, Carbonate, wie Natriumdicarbonat und andere aus dem Stand der Technik bekannte Schäumungsmittel."

Figur 1a zeigt anhand eines Referenzbeispiels zum Funktionsprinzip der erfindungsgemäßen Kapsel zwei getrennte Kapselelemente (1) und (2) mit den vorgefertigten Löchern (6) und (7) vor dem Ineinanderstecken. In dem hier dargestellten Beispiel wird beim teleskopartigen Ineinanderschieben die Kapselkappe (1) auf den Kapselkörper (2) aufgesetzt. (Auch der umgekehrte Fall des Einschiebens der Kapselkappe (1) in den Kapselkörper (2) hinein ist denkbar; für diesen Fall müssten alle nachfolgenden Beziehungen bzgl. "innen" und "außen" gerade umgekehrt betrachtet werden). Für die hier für die Darstellung gewählte Form mit Aufsetzen der Kapselkappe (1) auf den Kapselkörper (2) ist der äußere Durchmesser der Kapselkappe (1) im Bereich ihrer Becheröffnung etwas größer als der

Kapselkörper (2). Der äußere Durchmesser des Kapselkörpers (2) ist an dieser Stelle von vergleichbarer Größe wie der innere Durchmesser der Kapselkappe (1), wobei die Durchmesser von ihren Toleranzen her so aufeinander abgestimmt sind, dass die Kapselelemente beim Fügen ohne nennenswerte Spaltbildung im Bereich der Kapselmantelbereiche ineinander passen. In der gewählten Darstellung bildet somit nach Ineinanderstecken der Kapselelemente der Kapselkörper (2) die innere Wand der Kapsel im Bereich der zwei aufeinander liegenden Mantelbereiche der Kapselelemente.
Die Kapsel wird mit der pulverförmigen Arzneimittelzubereitung gefüllt - z.B. durch Einfüllen der Zubereitung in den Kapselkörper - und nach der Füllung wird die Kapselkappe (1) bis zu einer ersten Einsteckstellung auf den Kapselkörper (2) aufgeschoben. Der mit "p" gekennzeichnete Pfeil in den Abbildungen kennzeichnet die Richtung, in der die Kapselelemente zusammengeschoben werden bzw. zusammengeschoben worden sind. Er soll des Weiteren den Druck symbolisieren, der für dieses Zusammenschieben angewendet werden muss.
In der ersten - in Figur 1b gezeigten - Einsteckstellung greifen Rastelemente am äußeren Mantelbereich des Kapselkörpers (2) und Rastelemente am inneren Mantelbereich der Kapselkappe (1) ineinander. In allen hier dargestellten Figuren sind diese Rastelemente in Form von ringförmig umlaufenden Vorsprüngen oder Wülsten und Nuten oder Sicken dargestellt. So hat der Kapselkörper (2) in Figur 1 beispielsweise eine nach außen gerichtete umlaufende Wulst (5), die in der ersten Einsteckstellung in eine erste ringförmig umlaufende Sicke (4) innen an der Kapselkappe (1) einrastet. Die gleiche Wirkung würde erzielt durch eine ringförmige Nut im äußeren Mantelbereich des Kapselkörpers (1), wobei in der Einsteckstellung ein ringförmig umlaufender Vorsprung am inneren Mantelbereich der Kapselkappe (1) in diese Nut einrastet. Die Rastelemente müssen jedoch nicht zwangsläufig ringförmig ausgebildet sein, sondern können auch durch eher punktförmige Erhebungen in Kapselkörper und passende Vertiefungen in Kapselkappe bzw. umgekehrt gebildet werden. In einer bevorzugten Ausführungsform weist der Kapselkörper mehrere punktförmige, ringförmig angeordnete Erhebungen auf, die in eine entsprechende, vorzugsweise ringförmig umlaufende, Nut außen an der Kapselkappe einrasten. Bezüglich der Auslegung einer solchen eher punktuell gebildeten Verrastung sei hiermit als Beispiel auf die Offenbarung der WO2006/074982 A2 auf Seite 7 Zeile 1 bis Seite 8 Zeile 32 verwiesen.

In der ersten Einsteckstellung (siehe Figur 1b) überlappen sich die Mantelbereiche von Kapselkörper (2) und Kapselkappe (1) vorzugsweise so, dass der Mantel des Kapselkörpers (2) das Loch (6) in der Kapselkappe (1) von innen abdeckt und der Mantel der Kapselkappe (1) das Loch (7) im Kapselkörper (2) von außen abdeckt. Die Kapsel ist in dieser ersten Einsteckstellung komplett geschlossen.

In der zweiten - in Figur 1c gezeigten - Einsteckstellung sind Kapselkappe (1) und Kapselkörper (2) soweit ineinander geschoben, dass die jeweiligen Löcher (6) und (7) einander überdecken. Die Kapsel ist in dieser zweiten Einsteckstellung also "geöffnet" in dem Sinne, dass Pulver aus dem Inneren der Kapsel ausgebracht werden kann. Das vorgefertigte Loch (6) im hier außen liegenden Mantelbereich der Kapselkappe (1) ist größer als das Loch (7) des innen liegenden Mantelbereichs des Kapselkörpers (2). Dadurch dass eines der beiden Löcher kleiner als das andere ist, wird sichergestellt, dass auch bei Unregelmäßigkeiten beim Ineinanderstecken von Kapselkappe (1) und Kapselkörper (2) es nicht zur teilweisen Abdeckung des für die Ausbringung des Pulvers vorgesehenen Lochdurchmessers kommt. Die Größe des innenliegenden Loches (7) bestimmt in diesem bevorzugten Fall die Gesamtgröße des Lochs in der zusammengeschobenen Kapsel. Auf diese Weise kommt es auf der Innenseite der Kapsel nicht innen am Loch zu einer Stufe, an der bei Ausbringung pulverförmiges Material hängen bleiben könnte. Für die zweite Einsteckstellung sind an der Kapselkappe (1) ähnliche Rastelemente wie für die erste Einsteckstellung vorgesehen. Dementsprechend weist die Kapselkappe (1) im Darstellungsbeispiel von Figur 1 eine zweite Sicke (3) auf, in welche die Wulst (5) oder ein anderes vorspringendes Rastelement am inneren Mantelbereich des Kapselkörpers (2) einrasten kann. Dadurch werden die beiden Löcher (6) und (7) in ihrer Überdeckung gehalten und die Kapselelemente können sich auch bei Bewegung der Kapsel nicht mehr relativ zueinander verschieben. Die für die erste und zweite Einsteckstellung benötigten Rastelemente können beispielsweise durch Formgestaltung im Spritzguss von Kapselkappe (1) und Kapselkörper (2) oder durch Materialverformung an den Bauteilen realisiert werden. So kann beispielsweise eine innen im Kapselkörper umlaufende Wulst (5) mit einer außen umlaufenden Sicke einhergehen.

Zusätzlich zu der beschriebenen - als makroskopisch anzusehenden - Strukturierung der Kapselelemente weist eine weitere Ausführungsform der erfindungsgemäßen Kapsel eine Mikro- oder Nanostruktur oder Oberflächenbeschichtung innen an einem Kapselelement auf.

Dies ist insbesondere jenes Kapselelement, das bei aneinanderliegenden Kapselmänteln die Außenwand der Kapsel bildet - die Kapselkappe (1) im Beispiel von Figur 1. Die Mikrostruktur befindet sich hierbei vorzugsweise innen an der dem anderen Kapselelement zugewandten Mantelfläche. Insbesondere erstreckt sich die Mikrostruktur über einen in einem ringförmigen Bereich der inneren Mantelfläche, wobei dieser ringförmige Bereich beim Vorliegen der Kapsel in der ersten Einsteckstellung (Figur 1a) eine direkte Wand des Hohlraums der Kapsel bildet und beim Vorliegen der Kapsel in der zweiten Einsteckstellung (Figur 1c) an der äußeren Mantelfläche des anderen Kapselelementes - des Kapselkörpers (2) im Beispiel von Figur 1 - anliegt.
Diese Mikrostruktur bewirkt einen so genannten Lotus-Effekt, d.h. dass sie die Anhaftung von bestimmtem Material an dieser Oberfläche reduziert. Um den optimalen Effekt zu erzielen muss die Art der Mikrostruktur so gewählt sein, dass sie die geringsten Hafteigenschaften für die definierte pharmazeutische Zubereitung bietet, die in dem entsprechenden Kapseltyp bevorratet werden soll. Auf diese Weise haftet kein oder nur sehr wenig Material aus der pharmazeutischen Zubereitung - beispielsweise Pulver - an der Innenwand der Kapsel an. Dies hat insbesondere in dem beschriebenen ringförmigen Bereich den Effekt, dass es beim Zusammenschieben der Kapsel von der ersten zur zweiten Einsteckstellung nicht zu Reibung durch an der Wand anhaftendes Material kommt. Eine Ausdehnung der Mikrostruktur auf alle inneren Wandbereiche der Kapsel ist ebenfalls möglich und hat den Effekt, dass kein Material durch Wandanhaftung in der Kapsel verbleibt, wenn das Material während eines Zerstäubungsvorgangs ausgebracht wird.
Die Mikrostruktur wird durch Erhebungen und/oder Vertiefungen in der Oberfläche gebildet. Die Erhebungen und/oder Vertiefungen können die Form von Spitzen, Halbkugeln, planaren Flächen, Keilen etc. haben. Sie können eine zufällige Anordnung aufweisen oder geordnet sein, z.B. in Reihen, Kreisen, zick-zack-förmig, meanderförmig etc.
Der Abstand zwischen den Erhebungen der Oberflächenstruktur liegt im Bereich von 0,1 bis 200 Mikrometer, vorzugsweise 0,1 bis 100 Mikrometer. Bei pulverförmigen pharmazeutischen Zubereitungen sind hierbei Strukturdimensionen bevorzugt, die kleiner sind als die Korngrößen des Pulvers. Am stärksten bevorzugt sind Höhen von Erhebungen bzw. Tiefen von Vertiefungen im Bereich von 0,1 bis 50 Mikrometer und Abstände von 0,1 bis 10 Mikrometer.
Bezüglich von Verfahren zur Erzeugung einer solchen Mikrostruktur und ihrer Eigenschaften sei hiermit auf die Offenbarung der WO2004/062716 A1 auf Seite 11 Zeile 1 bis Seite 13 Zeile 13 verwiesen.
Bevorzugt werden für die Anbringung einer Mikrostruktur an Kapselinnenwänden solche Verfahren, die kein zusätzliches Material in die Kapseln hinein bringen, d.h. solche Mikrostrukturen, wie sie allein in dem das jeweilige Kapselelement bildenden Material abgeformt werden können. Im bevorzugten Fall von Kapselelementen, die in Spritzgussverfahren hergestellt werden, sind die Mikrostrukturen vorzugsweise bereits in den jeweiligen Formeinsätzen der Spritzgusswerkzeugen - sozusagen spiegelbildlich - abgebildet, so dass die Kapselelemente diese Mikrostrukturen wie auch die vorgefertigten Löcher bereits im ersten Fertigungsschritt erhalten. Alternativ können solche Mikrostrukturen an den Kapselinnenwänden auch durch nachträgliches Prägen - beispielsweise durch einen aufspreizbaren Stempel, der in die Hauptöffnung des Kapselelements eingeführt wird - oder durch subtraktive Oberflächenbehandlung wie Ätzen oder galvanische Abtragung geschaffen werden.

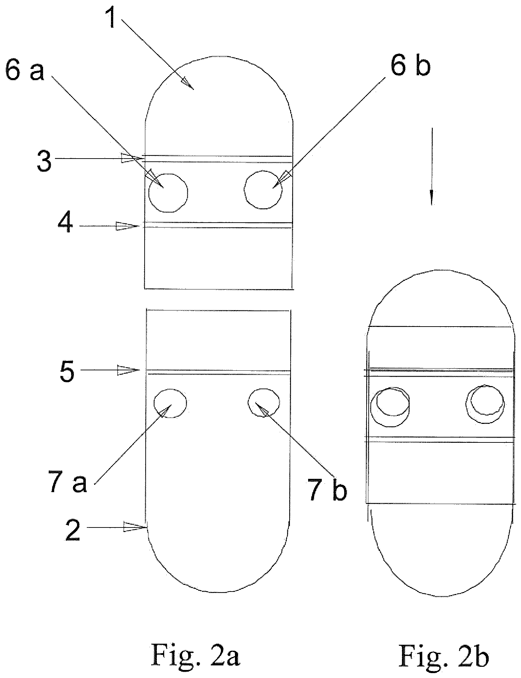

Figur 2 zeigt anhand eines zweiten Referenzbeispiels zum Funktionsprinzip der erfindungsgemäßen Kapsel eine weitere aus einer Kapselkappe (1) und einem Kapselkörper (2) zusammengesetzten Kapsel. Dieses Beispiel unterscheidet sich vom ersten nur dadurch, dass erfindungsgemäß Kapselkappe (1) und Kapselkörper (2) jeweils mehrere vorgefertigte Löcher (6a) und (6b) bzw. (7a) und (7b) aufweisen. In der nicht gezeigten ersten Einsteckstellung sind alle diese Löcher durch das jeweils andere Kapselelement analog zum ersten Ausführungsbeispiel verschlossen. In der zweiten Einsteckstellung (Figur 2b) überdecken sich die jeweiligen Löcher (6a) und (6b) der Kapselkappe (1) mit den Löchern (7a) und (7b) des Kapselkörpers (2), so dass die Kapsel an mehreren Stellen geöffnet ist. Im Darstellungsbeispiel sind jeweils zwei Löcher (6a, 6b) bzw (7a, 7b) in Kapselkappe (1) und Kapselkörper (2) abgebildet, die in zwei Öffnungen an der Kapsel in der zweiten Einsteckstellung resultieren. Auf diese Weise können jedoch beliebig viele Löcher vorgesehen werden. Die Löcher können je nach Gestaltung der Mantelbereiche von Kapselkappe (1) und Kapselkörper (2) über Umfang und Länge der Kapsel verteilt werden. Die resultierende geöffnete Kapsel weist erfndungsgemäß zwei Löcher auf, die sich auf dem Mantelbereich der Kapsel jeweils nahe der entgegengesetzten Enden der Kapsel befinden, also bezogen auf die Länge der Kapsel deutlich voneinander beabstandet sind.

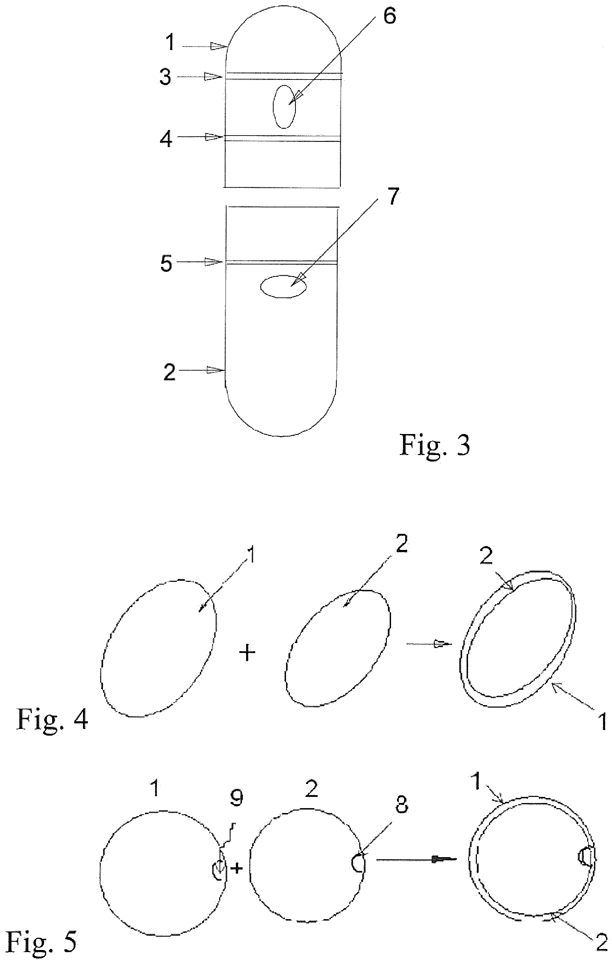

Figur 3 zeigt anhand eines dritten Referenzbeispiels zum Funktionsprinzip der erfindungsgemäßen Kapsel eine aus einer Kapselkappe (1) und einem Kapselkörper (2) zusammensetzbaren Kapsel. Dieses Beispiel unterscheidet sich von dem ersten nur dadurch, dass die Löcher (6) und (7) in Kapselkappe (1) und Kapselkörper (2) statt einer kreisförmigen eine elliptische Gestalt haben. Die Längsachsen dieser Ellipsen sind relativ zueinander um 90° verdreht. Auf diese Weise wird bei Unregelmäßigkeiten beim Zusammenschieben sichergestellt, dass die bei der Überdeckung zustande kommenden Öffnungen immer eine gleichbleibende Gesamtfläche einnehmen. Statt einer elliptischen Lochform können an dieser Stelle auch entsprechend ausgerichtete Langlöcher verwendet werden.

Figur 4 und 5 zeigen Kapselkörper (2) und Kapselkappe (1) schematisch im Querschnitt vor und nach dem Ineinanderstecken für verschiedene Ausführungsformen. Damit beim Ineinanderstecken von Kapselkappe (1) und Kapselkörper (2) die jeweiligen Löcher (6) und (7) sauber zur Überdeckung gebracht werden können, müssen die Kapselelemente bezogen auf ihren Umfang zueinander ausgerichtet ineinandergesteckt werden. Dies erfordert eine Struktur und Gegenstruktur ähnlich eines Schlüssel-Schloss-Prinzips an den Kanten bzw. jeweiligen Mantelflächen von Kapselkappe (1) und Kapselkörper (2). Des Weiteren ist eine Längsführung von Vorteil, die bewirkt, dass es nach gesteuertem erstem Ineinanderstecken der Kapselelemente zu keiner unerwünschten Verdrehung der Kapselelemente zueinander mehr kommen kann. In dem Ausführungsbeispiel gemäß Figur 4 sind Kapselkappe (1) und Kapselkörper (2) gleichermaßen mit leicht elliptischem Querschnitt gestaltet. Die Ellipsenform erzwingt hierbei das orientierte Ineinanderstecken der beiden Kapselelemente. Bei der Zusammenführung der elliptischen Kapselelemente ergeben sich folglich zwei mögliche, um 180° zueinander verdrehte Orientierungen. Vorzugsweise sollte daher eines der beiden Kapselelemente, besonders bevorzugt das äußere (in diesem Beispiel also die Kapselkappe (1)), nicht nur das mindestens eine vorgefertigte Loch (6) aufweisen sondern auch eine Duplizierung der Lochstruktur um 180° entlang des Umfangs des Kapselelements versetzt aufweisen. Somit wird unabhängig von der Orientierung beim Zusammenführen zweier elliptischer Kapselelemente immer ein Loch (6) beim Ineinanderschieben in die zweite Einsteckstellung freigegeben.
In dem Ausführungsbeispiel gemäß Figur 5 weist der Außenbereich des Kapselkörpers (2) zusätzlich zu den nicht gezeigten Rastelementen einen parallel zur Längsachse verlaufenden Steg (8) auf, dessen Kontur zu einer ebenfalls längs, innen an der Kapselkappe (1) angebrachten Nut (9) passt. Die Kapselelemente lassen sich nur in der azimutalen Ausrichtung, in welcher der Steg (8) und die Nut (9) ineinander greifen, zusammen schieben. Steg (8) und Nut (9) bilden eine Führung über die gesamte Einschublänge. Wahlweise können auch mehrere unterschiedlich zueinander beabstandete und/oder unterschiedliche breite Steg- und-Nut-Paare an den Mantelflächen der Kapselelemente angeordnet sein.
In einer nicht dargestellten Ausführungsform kann das gezeigte Strukturpaar aus Steg (8) und Nut (9) auch eine Kurvenbahn aufweisen, durch welche ein Zusammenschieben der Kapselelemente eine Drehung derselben relativ zueinander erzwingt. Dies kann insbesondere bei dem gezielten Verdecken und Freilegen von mehreren Löchern in der Kapsel von Vorteil sein.

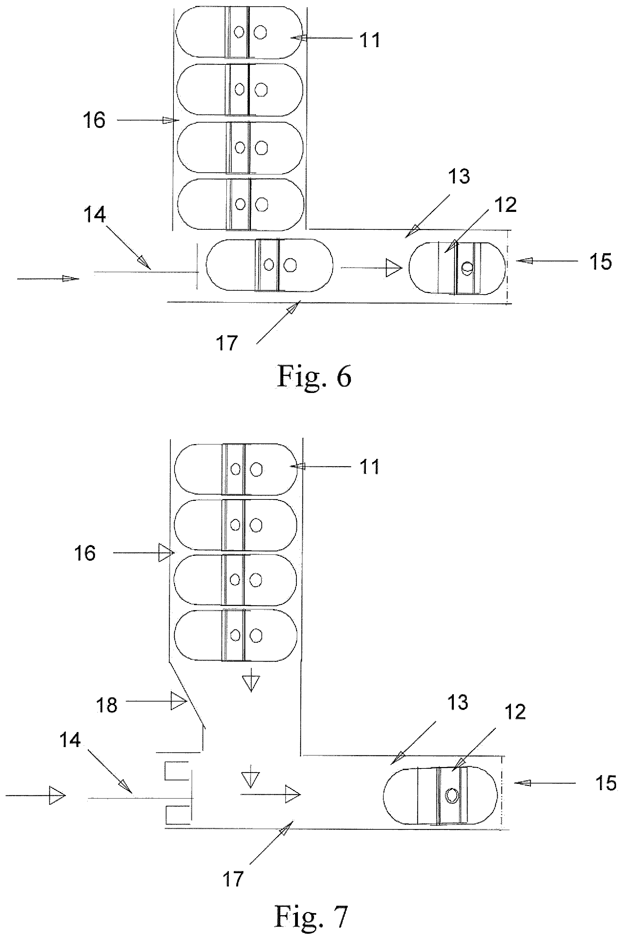

Figur 6 zeigt schematisch die Funktionsweise eines Inhalators, in dem mehrere Kapseln (hier in Referenzform skizziert) mit vorgefertigten Löchern bevorratet sind. In dieser Ausführungsform sind mehrere Kapseln (11) in erster Einsteckstellung in einem Magazin bevorratet (Die Darstellung der Kapsel (11) in erster Einsteckstellung ist in Fig. 6,7 und 8 bezüglich der Lochpositionen nicht funktionsgetreu und daher hier nur rein schematisch zu verstehen). Das Magazin ist in Form eines vertikalen Schachts (16) dargestellt, dessen Querschnitt an die Längsausdehnung der Kapsel (11) angepasst ist, d.h. beispielsweise rechteckig oder langlochförmig mit Breiten nur wenig größer als Länge und Durchmesser der Kapsel (11). Die Kapseln, die sich in ihrem ersten Verrastungszustand befinden, sind zunächst im Magazin gestapelt. Die jeweils zur Anwendung kommende Kapsel (11) wird in einen Kanal (17) gefördert, entlang dessen vorzugsweise auch die im Inhalator wirkende Luftströmung verläuft. Diese Förderung kann entweder allein auf der Wirkung der Schwerkraft beruhen oder durch eine oben am Magazin angebrachte Federkraft unterstützt werden. In dem Kanal (17) wird die Kapsel (11) durch einen Schieber (14) unmittelbar vor der Anwendung des Inhalators in die zweite Einsteckstellung zusammengeschoben, so dass sich die jeweiligen Löcher von Kapselkappe und Kapselkörper übereinander befinden. Die exakte Zusammenführung wird durch entsprechende Dimensionierung gesichert. Der Schieber (14) kann eine Lufteintrittsöffnung aufweisen und somit die Wand der Kapselkammer (13) bilden, in der das Pulver im Luftstrom aus der Kapsel (12) ausgebracht werden soll. Auf diese Weise kann unmittelbar nach der Kapselöffnung die Inhalation des Kapselinhaltes erfolgen. Dabei muss der Schieber (14) durch entsprechende Konstruktion so arretiert werden, dass er zusammen mit der Begrenzung der Kapselkammer (13) in Schubrichtung eine Kapselkammer (13) bildet, deren Länge derart auf die Länge der Kapsel (12) in ihrer zweiten Einsteckstellung angepasst ist, dass sich die Kapsel (12) gemäß dem Bernoulli-Effekt im Luftstrom bewegen und vibrieren kann. Der Schieber (14) kann somit mindestens drei Positionen im Kanal (17) einnehmen: Eine erste Position, in der eine Kapsel (11) aus dem Magazin in den Kanal (17) gefördert werden kann, eine zweite, ganz in den Kanal (17) eingeschobene Position, in der die Kapsel in ihre zweite Einsteckstellung zusammen geschoben wird (gleichzeitig stößt sie an die Begrenzung der Kapselkammer (13) in Richtung Luftauslass), und eine dritte, gegenüber der zweiten leicht zurückgezogene Position, in der der Schieber die Lufteinlass-seitige Begrenzung der Kapselkammer (13) während der Zerstäubung bildet. Die Begrenzung der Kapselkammer (13) in Richtung des Luftauslasses wird vorzugsweise durch ein Sieb (15) oder durch ein aerodynamisch vorteilhaft geformtes Bauteil gebildet, das nur einen geringen Strömungswiderstand darstellt. An diese den Luftauslass beinhaltende Begrenzung der Kapselkammer (13) schließt sich ein nicht dargestelltes Mundstück an, an dem der ebenfalls nicht dargestellte Anwender inhaliert und dadurch den zur Zerstäubung des Kapselinhalts notwendigen Luftstrom erzeugt.
Um nach Ausbringung der pharmazeutischen Zubereitung aus der Kapsel (12) die Kapselkammer (13) wieder mit einer neuen Kapsel (11) bestücken zu können, ist vorzugsweise eine Möglichkeit zum Öffnen der Kapselkammer (13) zur Entnahme der gebrauchten Kapsel (12) am Inhalator angebracht. Diese Öffnungsmöglichkeit kann beispielsweise durch eine verschließbaren Klappe in der Wand des Kanals (17) auf der dem Schacht (16) abgewandten Seite der Kapselkammer (13) gebildet werden.

Figur 7 zeigt schematisch die Funktionsweise einer weiteren Ausführungsform eines Inhalators, in dem mehrere Kapseln (hier in Referenzform skizziert) mit vorgefertigten Löchern bevorratet sind. In dieser Ausführungsform sind mehrere Kapseln (11) in erster Einsteckstellung in einem schachtförmigen Magazin bevorratet. In dieser Ausführungsform weist der Schacht (16) an seinem Ausgang zum Kanal (17) bzw. zur Kapselkammer (13) eine Verjüngung auf, wobei eine Wand in diesem Verjüngungsbereich als schiefe Ebene oder Rampe oder vorzugsweise als konische Führungsschiene (18) ausgebildet ist. Der Schachtdurchmesser am Ende der Verjüngung bzw. am Schachtausgang entspricht der Größe der Kapsel (12) in zweiten Einsteckstellung, d.h. im vollständig ineinander geschobenen Zustand. Durch oben am Schacht (16) angelegten Druck auf die Kapseln (11) - der Druck wird hierbei von Kapsel (11) zu Kapsel (11) von oben nach unten übertragen - erzwingt die gleichmäßige Verkleinerung des Schachtdurchmessers eine Verkleinerung der Kapsellänge, d.h. die schiefe Ebene oder Rampe oder konische Führungsschiene (18) bewirkt ein weiteres Ineinanderschieben von Kapselkörper (2) und Kapselkappe (1) bis am Schachtausgang die Kapsel (2) in der zweiten Einsteckstellung vorliegt.
Im Darstellungsbeispiel in Figur 7 ist unabhängig von dem Zusammenschieben der Kapsel (11) am Ende des Schachts (16) ein Schieber (14) im Kanal (17) angeordnet. Dieser Schieber (14) hat hier vor allem die Aufgabe, die Kapsel (12) in die Kapselkammer (13) zu schieben. Andere Transportmöglichkeiten von Magazin zu Kapselkammer (13) sind ebenfalls möglich, z.B. über Klappen in der Wand der Kapselkammer (13). Der Schieber (14) in diesem Ausführungsbeispiel nimmt vor allem zwei Positionen ein: Eine erste Position, in der die bereits zusammengeschobene Kapsel (12) aus dem Magazin in den Kanal (17) gefördert werden kann, und eine weitere Position, in der der Schieber die Lufteinlass-seitige Begrenzung der Kapselkammer (13) während der Zerstäubung bildet und die Kapsel (12) in der Kapselkammer (13) Spielraum für Vibration und/oder Bewegung hat.

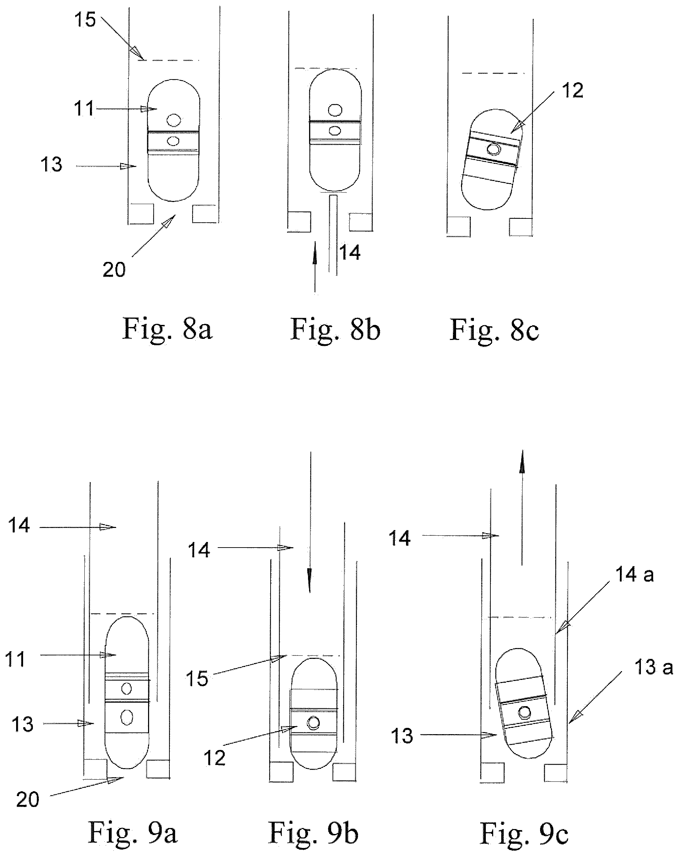

Die Figuren 8, 9 und 10 zeigen anhand verschiedener Ausführungsformen schematisch die Funktionsweise eines Inhalators, in dem sich eine Kapsel mit vorgefertigten Löchern bereits in einer Kapselkammer (13) befindet. Die hier gezeigten Funktionsweisen sind zwar jeweils auch auf die befüllte Kapselkammer eines Inhalators mit mehreren bevorrateten Kapseln anwendbar, in erster Linie beziehen sich die hier gezeigten Ausführungsformen jedoch auf Inhalatoren zur einmaligen Anwendung. Die hier vorgestellten Inhalatoren bestehen vom Prinzip her lediglich aus einer Kapselkammer (13) mit voreingesetzter Kapsel (11), einem gegebenenfalls mittels einer Feder (19) rückstellbaren Schieber (14) und einem Mundstück mit Inhalationskanal, an den ein Luftauslass aus der Kapselkammer (13) angeschlossen ist. Die Kapselkammer (13) weist neben besagtem Luftauslass einen Lufteinlass auf. Die Begrenzung der Kapselkammer (13) wird vorzugsweise durch ein Sieb (15) oder ein aerodynamisch vorteilhaft geformtes Bauteil oder Element gebildet, das nur einen geringen Strömungswiderstand darstellt.
In Figur 8a ist eine solche Kapselkammer (13) mit voreingesetzter Kapsel (11) in der ersten Einsteckstellung zu sehen. Die Kapselkammer (13) weist eine Lufteinlassöffnung (20) auf, durch die in Figur 8b ein Stößel oder Schieber (14) eingeführt wird, mit dem die Kapsel (11) gegen das Sieb (15), d.h. gegen die Begrenzung der Kapselkammer (13) auf der Seite des Luftauslasses geschoben wird. Durch weiter in Richtung des Luftauslasses der Kapselkammer (13) gerichteten Druck werden Kapselkappe (1) und Kapselkörper (2) weiter ineinander geschoben, bis die Kapsel (12) bis zu ihrer zweiten Einsteckstellung zusammengeschoben ist. Anschließend (Figur 8c) wird der Stößel oder Schieber (14) wieder aus der Kapselkammer (13) herausgezogen und die zusammengeschobene Kapsel (12) steht zur Inhalation bereit: Ihre Löcher sind zur Ausbringung des vorzugsweise pulverförmigen, innenliegenden
Materials geöffnet, und sie hat in der Kapselkammer (13) Bewegungsspielraum für Vibration und/oder Bewegung. In der in Figur 8a gezeigten Situation der voreingesetzten Kapsel (11) in erster Einsteckstellung ist kein Bewegungsspielraum für die Kapsel notwendig, bzw. sollte so klein wie möglich gehalten werden, um ein zufälliges Verkippen der Kapsel beim Einschieben des Stempels oder Schieber (14) zu vermeiden.

Figur 9 zeigt einen Inhalator, in dem der Schieber (14) einen Teil der Kapselkammer (13) bildet, in den der Luftauslass integriert ist. Der Schieber (14) umfasst dabei die Begrenzung der Kapselkammer (13) auf der Seite des Luftauslasses. Zusätzlich kann er - wie in Figur 9 dargestellt - auch teleskopartig einen Teil der Seitenwand der Kapselkammer (13) bilden; Ausgestaltungen ohne dieses Merkmal sind aber ebenfalls möglich.
Figur 9a zeigt eine vorzugsweise im Wesentlichen zylindrische Kapselkammer (13) mit voreingesetzter Kapsel (11) in erster Einsteckstellung. Die Lufteinlassöffnung (20) ist hier mittig in der dem Luftauslass entgegengesetzten Seite der Kapselkammer (13), d.h. - bezogen auf die Abbildung - unten in der Kapselkammer (13). Vorzugsweise ist die Position des Schiebers (14) in dieser Situation der voreingesetzten Kapsel (11) so, dass die Kapsel (11) oben in der Kapselkammer (13) an deren Begrenzung, d.h. bevorzugt an einem Sieb (15) oder einem aerodynamischen Element, anliegt und unten mit ihrem abgerundeten Endbereich so in die Lufteinlassöffnung (20) eintaucht, dass sie diese verschließt. Auf diese Weise kann z.B. beim Transport des Inhalators nach Entnahme aus einer Umverpackung vermieden werden, dass Verunreinigungen in die Kapselkammer gelangen.
In Figur 9b wird die Kapsel (11) mittels des Schiebers (14), genauer gesagt mit der zu ihm gehörenden Begrenzung der Kapselkammer (13), so gegen ihre Auflage im Bereich des Lufteinlasses gedrückt, dass Kapselkappe und Kapselkörper weiter ineinander geschoben werden bis die zweite Einsteckstellung der Kapsel (12) erreicht ist. In diesem Beispiel hat der Schieber (14) nicht nur eine Schubfläche in Richtung der Kapsel (11) sondern auch in die Kapselkammer (13) hineinragende Wandbereiche. Die Kapselkammer (13) wird hier gewissermaßen aus zwei mit den Öffnungen ineinander gesteckten Hohlzylindern gebildet, wodurch sie seitlich eine doppelte Wandung erhält, die aus der eigentlichen Längswand (13a) der Kapselkammer (13) und der inneren eingeschobenen Längswand (14a) des Schiebers (14) gebildet wird. Hierbei muss die Länge der eingeschobenen Längswand (14a) derart auf die Länge der Kapsel (12) in der zweiten Einsteckstellung abgestimmt sein, dass der Schieber (14) entsprechend weit genug in die Kapselkammer (13) eingeschoben werden kann. Nach Zusammenschieben der Kapsel (12) muss der Schieber (14) wieder - z.B. in seine Ausgangsposition - zurück gezogen und so arretiert werden, dass die Länge der Kapselkammer (13) für die Bewegung der Kapsel (12) bei der Inhalation fest fixiert und somit auch definiert ist. Vorzugsweise ist für die Vermeidung von Nebenluft während der Inhalation eine gleitende Dichtung zwischen der eingeschobenen Längswand (14a) des Schiebers (14) und der sie aufnehmenden Längswand (13a) der Kapselkammer (13) ausgebildet bzw. eingesetzt.

Vorzugsweise besteht der Inhalator gemäß der in Figur 9 gezeigten Funktionsweise neben der Kapsel (11) aus zwei Bauteilen, die zum Freigeben der Löcher der Kapsel (11) teleskopartig ineinander und zur anschließenden Anwendung des Inhalators wieder auseinander gezogen werden: Einem Bauteil, das den Teil der Kapselkammer (13) mit dem Lufteinlass beinhaltet, und einem weiteren Bauteil, das den Teil der Kapselkammer (13) mit dem Luftauslass, den Inhalationskanal und vorzugsweise auch das daran angeschlossene Mundstück beinhaltet. Bevorzugt sind beide Bauteile einstückig z.B. aus Kunststoffspritzguss gefertigt. Je nach Ausgestaltung der Griffflächen außen am Inhalator kann jedes der beiden Bauteile in diesem Ausführungsbeispiel als "Schieber" bezeichnet werden: Entweder der Inhalator ist so gestaltet, dass er unten gehalten wird und das Mundstück - wie in Figur 9b skizziert - hinein gedrückt wird, oder er ist so gestaltet, dass man ihn an einem das Mundstück umfassenden Hauptkörper hält und das andere Bauteil hineindrückt.

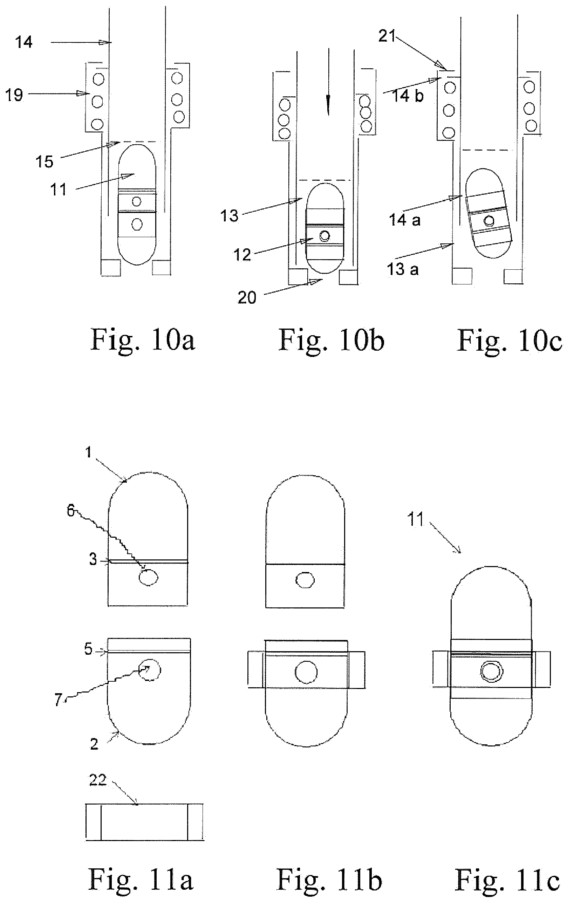

Figur 10a, 10b und 10c zeigen einen Inhalator entsprechend dem in den Figuren 9a, 9b und 9c, wobei sich der Inhalator in Figur 10 lediglich dadurch vom vorherigen unterscheidet, dass er eine Feder (19) umfasst, die eine Rückstellung der beiden Bauteile nach ihrem Zusammenschieben ineinander bewirkt. In diesem Ausführungsbeispiel weist der Schieber (14) einen äußeren Vorsprung (14b) auf, mit dem die Feder (19) beim Schieben des Schiebers (14) in die Kapselkammer (13) gegen die Federkraft zusammen gedrückt wird. Wird der äußere Druck auf den Schieber (14) beendet, drückt die Feder (19) den Schieber (14) wieder über seinen Vorsprung (14b) nach außen und zwar gerade so weit wie es eine Anschlagkante (21) zulässt, welche zur Halterung der Feder (19) im Inhalator gehört und die maximale Ausdehnung der Feder (19) begrenzt.

Figur 11 zeigt in schematischer Darstellung eine alternative (hier nicht erfindungsgemäße) Kapsel bestehend aus Kapselkappe (1), Kapselkörper (2) und einem Ring (22). Kapselkappe (1) und Kapselkörper (2) haben analog zu dem Beispiel von Figur 1 beide ebenfalls eine becherartige Form und können teleskopartig über ihre Öffnungen ineinander gesteckt werden, wobei diesbezüglich anhand von Figur 1 beschriebene Aspekte hier ebenfalls ihre Gültigkeit haben. Aus Gründen der einfacheren Befüllung ist im Darstellungsbeispiel von Figur 11 abweichend zu den anderen Darstellungsbeispielen eine Form gewählt worden, bei der die Kapselkappe (1) in den Kapselkörper (2) eingeschoben wird (Ausführungsformen mit außen aufgeschobener Kappe sind aber ebenfalls möglich). Der Kapselkörper (2) mit gegenüber der Kapselkappe (1) vergrößertem Loch (7) bildet hier also die äußere Wand der Kapsel im Bereich der zwei aufeinander liegenden Mantelbereiche der Kapselelemente, die Kapselmantelbereiche der beiden Kapselelemente weisen analog zu den vorherigen Beispielen miteinander wechselwirkenden Strukturierungen auf.
Figur 11a zeigt die beiden getrennten Kapselelemente (1) und (2) mit den vorgefertigten Löchern (6) und (7) vor dem Ineinanderstecken und vor Zusammenfügen mit dem Ring (22). Figur 11b zeigt den Kapselkörper (2) nach Zusammenfügen mit dem Ring (22). Der Ring (22) verdeckt hierbei das Loch (7) des Kapselkörpers (2). Dies ist vorzugsweise die Situation, in der die pharmazeutische vorzugsweise pulverförmige Zubereitung in den Kapselkörper (2) eingefüllt wird, der dann - wie in Figur 11c gezeigt - mit der Kapselkappe (1) verschlossen wird. Die Kapselkappe (1) kann bei diesem Konzept direkt bis zum Erreichen der finalen Einsteckstellung in den Kapselkörper (2) geschoben werden.
Figur 11c zeigt die zusammengesetzte Kapsel (11), in der analog zum Referenzbeispiel von Figur 1 Kapselkappe (1) und Kapselkörper (2) mittels Rastelemente (3) und (5) miteinander verrastet sind. Die jeweiligen vorgefertigten Löcher (6) und (7) überdecken sich bei dieser zusammengesetzten Kapsel, das resultierende Loch wird in diesem Zustand jedoch vom Ring (22) verdeckt. Die Kapsel (11) kann so gelagert oder in einen geeigneten Inhalator ein gesetzt werden.

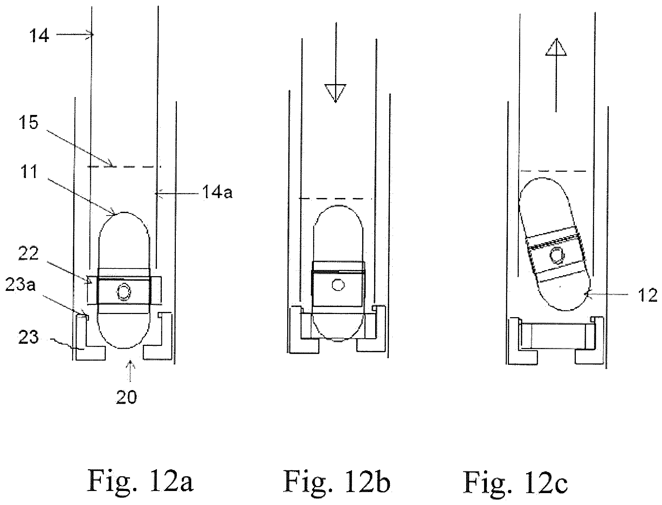

Die Figur 12 zeigt schematisch die Funktionsweise eines Inhalators, dessen Kapselkammer (13) bereits mit einer alternativen (nicht erfindungsgemäßen) Kapsel (11) befüllt ist, deren vorgefertigten Löcher gemäß des anhand des Beispiels gemäß Figur 11 geschilderten Prinzips über einen Ring von außen verschlossen sind. Ähnlich wie bei den in den Figuren 8, 9 und 10 gezeigten Funktionsweisen bezieht sich die hier gezeigte Ausführungsform bevorzugt auf einen Inhalator zur einmaligen Anwendung.
Figur 12 zeigt ähnlich zu Figur 9 einen Inhalator, in dem der Schieber (14) den Teil der Kapselkammer (13) bildet, in den der Luftauslass integriert ist. Der Schieber (14) umfasst dabei die Begrenzung der Kapselkammer (13) auf der Seite des Luftauslasses. Zusätzlich bildet der Schieber (14) teleskopartig einen Teil der Seitenwand der Kapselkammer (13). Der innere Durchmesser der sich so ergebenden eingeschobenen Längswand (14a) ist hierbei kleiner als der äußere Durchmesser des Rings (22), der die vorgefertigten Löcher (6) und (7) an der Kapsel (11) verschließt. Gleichzeitig ist der Durchmesser der eingeschobenen Längswand (14a) größer als der Durchmesser der im Wesentlichen zylinderförmigen Kapsel (12) ohne Ring (22).
In Figur 12b wird der Schieber (14) zum Lufteinlass hin bewegt, der sich in der Abbildung unten an der Kapselkammer (13) befindet. Hierbei stößt der untere Rand der eingeschobenen Längswand (14a) an den Ring (22). Dadurch wird vorzugsweise zunächst die ganze Kapsel (11) so nach unten gedrückt, dass sich das abgerundete untere Ende der Kapsel (11) so in die Lufteinlassöffnung (20) am Boden der Kapselkammer einfügt, dass die Kapsel sich insgesamt nach oben ausgerichtet und gleichzeitig sozusagen am unteren Anschlagspunkt anliegt (zusätzliches aber kein notwendiges Merkmal). Bei weiterem Druck auf den Schieber (14) von oben wird nun der Ring (22) auf dem äußeren Mantel der Kapsel (12) nach unten hin verschoben. Unten in der Kapselkammer (13) ist eine Aufnahme (23) für den Ring (22) ausgebildet oder eingesetzt. Die Unterkante der eingeschobenen Längswand (14a) des Schiebers (14) schiebt den Ring (22) in diese Aufnahme (23) über kleine Rastelemente (23a) hinweg, die den Ring (22) in seiner neuen Position in der Aufnahme (23) fixieren. In dieser Position des Rings (22) ist der Ring (22) von der Kapsel (12) getrennt worden und die übereinander liegenden vorgefertigten Löcher (6) und (7) liegen frei.
In einer anderen Variante (nicht in der Figur dargestellt) weist die Aufnahme (23) eine Nut auf, die den Ring (22) aufnimmt und einen Vorsprung an der Innenseite des Rings (22) bildet. Durch diesen Vorsprung wird die Kapsel (12) beim Eindrücken des Rings (22) in die Nut in Richtung Kapselkammer (13) vom Ring (22) gelöst. Der Vorsprung ist hierbei dann vorzugsweise so gestaltet, dass er analog zur Lufteinlassöffnung in der vorherigen Ausführungsform den unteren Anschlagspunkt für die Kapsel (12) in der Kapselkammer (13) bildet.
In Figur 12c wird der Schieber (14) wieder ein Stück in Richtung Luftauslass zurückgezogen, wodurch die Kapsel (12) Bewegungsspielraum für Vibration und/oder Bewegung erhält. Die Kapsel ist somit für die Ausbringung der innenliegenden pharmazeutischen Zubereitung vorbereitet.
In einer weiteren Variante ist am Schieber (14) analog zur Ausführungsform aus Figur 10 einer Feder (19) angeordnet, die eine Rückstellung des Schiebers nach Betätigung bewirkt.

Einige bevorzugte Bestandteile, Verbindungen und/oder Formulierungen der pharmazeutischen Zubereitung für den Einsatz in den erfindungsgemäßen Kapseln und/oder Inhalatoren werden im Folgenden aufgeführt. Die unten aufgelisteten Verbindungen können für sich genommen oder in Kombination untereinander in Zubereitungen eingesetzt werden.

In den unten aufgeführten Verbindungen ist W ein pharmakologisch, aktiver Wirkstoff und (beispielsweise) ausgewählt aus einer Gruppe bestehend aus Betamimetika, Anticholinergika, Corticosteroiden, PDE4-Inhibitoren, LTD4-Rezeptor (CysLT1, CysLT2, CysLT3) Antagonisten, EGFR-Hemmern, Dopamin-Agonisten, H1-Antihistaminika, PAF-Antagonisten, SYK-Inhibitoren, PDE3 Inhibitoren, Lipoxin A4 Derivate, FPRL1 Modulatoren, LTB4-Rezeptor (BLT1, BLT2) Antagonisten, Histamin H1 Rezeptor Antagonisten, Histamin H4 Rezeptor Antagonisten, PI3 Kinase Inhibitoren, Inhibitoren von Nicht-Rezeptor Tyrosine Kinasen wie zum Beispiel LYN, LCK, SYK, ZAP-70, FYN, BTK oder ITK, Inhibitoren von MAP Kinasen wie zum Beispiel p38, ERK1, ERK2, JNK1, JNK2, JNK3 oder SAP, Inhibitoren des NF-κB Signalwegs wie zum Beispiel IKK Kinase Inhibitoren, iNOS Inhibitoren, MRP4 Inhibitoren, Leukotriene Biosynthese Inhibitoren wie zum Beispiel 5-Lipoxygenase (5-LO) Inhibitoren, cPLA2 Inhibitoren, Leukotriene A4 Hydrolase Inhibitoren oder FLAP Inhibitoren, Nicht-steroidale antientzündliche Agenzien (NSAIDs), CRTH2 Antagonisten, DP1-Rezeptor Modulatoren, Thromboxane Rezeptor Antagonisten, Chemokine Rezeptor Antagonisten von CCR1, CCR2, CCR2A, CCR2B, CCR4, CCR5, CCR6, CCR7, CCR8, CCR9, CCR10, CCR11, CXCR1, CXCR2, CXCR3, CXCR4, CXCR5, CXCR6, CX3CR1, Neurokinin (NK1, NK2) Antagonisten, Sphingosine 1-Phosphat Rezeptor Modulatoren, Modulatoren von Adenosine Rezeptoren, Modulatoren von purinergen Rezeptoren wie zum Beispiel P2X7, Histone Deacetylase (HDAC) Aktivatoren, Bradykinin (BK1, BK2) Antagonisten, TACE Inhibitoren, Mucoregulatoren, PPAR gamma Agonisten, Rho Kinase Inhibitoren, interleukin 1-beta converting enzyme (ICE) Inhibitoren, Toll-Like Rezeptor (TLR) Modulatoren, HMG-CoA Reductase Inhibitoren, VLA-4 Antagonisten, ICAM-1 Inhibitoren, SHIP Agonisten, TNFα Antagonisten, GABAa Rezeptor Antagonisten, Immunotherapeutika, Substanzen gegen Schwellungen der Atemwege und Substanzen gegen Husten.

Weiterhin können zwei- oder dreifach Kombinationen von **W** gebildet werden. Beispielhaft genannte Kombinationen von **W** wären:
- **W** stellt ein Betamimetikum dar, kombiniert mit einem Anticholinergikum, Corticosteroid, PDE4-Inhibitor, EGFR-Hemmer oder LTD4-Rezeptor Antagonisten,
- **W** stellt ein Anticholinergikum dar, kombiniert mit einem Betamimetikum, Corticosteroid, PDE4-Inhibitor, EGFR-Hemmen oder LTD4-Rezeptor Antagonisten,
- **W** stellt ein Corticosteroid dar, kombiniert mit einem PDE4-Inhibitor, EGFR-Hemmer oder LTD4-Rezeptor Antagonist
- **W** stellt ein PDE4-Inhibitor dar, kombiniert mit einem EGFR-Hemmer oder LTD4-Rezeptor Antagonist
- **W** stellt ein EGFR-Hemmer dar, kombiniert mit einem Anticholinergikum.

Als Betamimetika gelangen hierbei vorzugsweise Verbindungen zur Anwendung, die ausgewählt sind aus der Gruppe bestehend aus Arformoterol, Carmoterol, Formoterol, Indacaterol, Salmeterol, Albuterole, Bambuterol, Bitolterol, Broxaterol, Carbuterol, Clenbuterol, Fenoterol, Hexoprenalin, Ibuterol, Isoetharin, Isoprenalin, Levosalbutamol, Mabuterol, Meluadrin, Metaproterenol, Milveterol, Orciprenalin, Pirbuterol, Procaterol, Reproterol, Rimiterol, Ritodrin, Salmefamol, Soterenol, Sulphonterol, Terbutalin, Tiaramid, Tolubuterol, Zinterol and
- 6-Hydroxy-8-{1-hydroxy-2-[2-(4-methoxy-phenyl)-1,1-dimethyl-ethylamino]-ethyl}-4H-benzo[1,4]oxazin-3-on
- 8-{2-[2-(2,4-Difluor-phenyl)-1,1-dimethyl-ethylamino]-1-hydroxy-ethyl}-6-hydroxy-4H-benzo[1,4]oxazin-3-on
- 8-{2-[2-(3,5-Difluor-phenyl)-1,1-dimethyl-ethylamino]-1-hydroxy-ethyl}-6-hydroxy-4H-benzo[1,4]oxazin-3 -on
- 8-{2-[2-(4-Ethoxy-phenyl)-1,1-dimethyl-ethylamino]-1-hydroxy-ethyl}-6-hydroxy-4H-benzo[1,4]oxazin-3-on
- 8-{2-[2-(4-Fluor-phenyl)-1,1-dimethyl-ethylamino]-1-hydroxy-ethyl}-6-hydroxy-4H-benzo[1,4]oxazin-3-on
- N-(5-{2-[3-(4,4-Diethyl-2-oxo-4H-benzo[d][1,3]oxazin-1-yl)-1,1-dimethyl-propylamino]-1-hydroxy-ethyl}-2-hydroxy-phenyl)-methansulfonamid
- N-(5-{2-[3-(4,4-Diethyl-6-fluoro-2-oxo-4H-benzo[d][1,3]oxazin-1-yl)-1,1-dimethyl-propylamino]-1-hydroxy-ethyl}-2-hydroxy-phenyl)-methansulfonamid
- N-(5-{2-[3-(4,4-Diethyl-6-methoxy-2-oxo-4H-benzo[d][1,3]oxazin-1-yl)-1,1-dimethyl-propylamino]-1-hydroxy-ethyl}-2-hydroxy-phenyl)-methansulfonamid
- N-(5-{2-[1,1-Dimethyl-3-(2-oxo-4,4-dipropyl-4H-benzo[d][1,3]oxazin-1-yl)-propylamino]-1-hydroxy-ethyl}-2-hydroxy-phenyl)-methansulfonamid
- 8-{2-[1,1-Dimethyl-3-(2-oxo-2,3-dihydro-benzoimidazol-1-yl)-propylamino]-1-hydroxyethyl}-6-hydroxy-4H-benzo[1,4]oxazin-3-on
- 8-{2-[1,1-Dimethyl-3-(6-methyl-2-oxo-2,3-dihydro-benzoimidazol-1-yl)-propylamino]-1-hydroxy-ethyl}-6-hydroxy-4H-benzo[1,4]oxazin-3-on
- 8-{2-[1,1-Dimethyl-3-(2-oxo-5-trifluormethyl-2,3-dihydro-benzoimidazol-1-yl)-propylamino]-1-hydroxy-ethyl}-6-hydroxy-4H-benzo[1,4]oxazin-3-on
- 8-{2-[1,1-Dimethyl-3-(3-methyl-2-oxo-2,3-dihydro-benzoimidazol-1-yl)-propylamino]-1-hydroxy-ethyl}-6-hydroxy-4H-benzo[1,4]oxazin-3-on
- N-[2-Hydroxy-5-((1R)-1-hydroxy-2-{2-[4-(2-hydroxy-2-phenyl-ethylamino)-phenyl]-ethylamino}-ethyl)-phenyl]-formamid
- 8-Hydroxy-5-((1R)-1-hydroxy-2-{2-[4-(6-methoxy-biphenyl-3-ylamino)-phenyl]-ethylamino}-ethyl)-1H-chinolin-2-on
- 8-Hydroxy-5-[(1R)-1-hydroxy-2-(6-phenethylamino-hexylamino)-ethyl]-1H-chinolin-2-on
- 5-[(1R)-2-(2-{4-[4-(2-Amino-2-methyl-propoxy)-phenylamino]-phenyl}-ethylamino)-1-hydroxy-ethyl]-8-hydroxy-1H-chinolin-2-on
- [3-(4-{6-[(2R)-2-Hydroxy-2-(4-hydroxy-3-hydroxymethyl-phenyl)-ethylamino]-hexyloxy}-butyl)-5-methyl-phenyl]-harnstoff
- 4-((1R)-2-{6-[2-(2,6-Dichlor-benzyloxy)-ethoxy]-hexylamino}-1-hydroxy-ethyl)-2-hydroxymethyl-phenol
- 3-(4-{6-[(2R)-2-Hydroxy-2-(4-hydroxy-3-hydroxymethyl-phenyl)-ethylamino]-hexyloxy}-butyl)-benzenesulfonamid
- 3-(3-{7-[(2R)-2-Hydroxy-2-(4-hydroxy-3-hydroxymethyl-phenyl)-ethylamino]-heptyloxy}-propyl)-benzenesulfonamid
- 4-((1R)-2-{6-[4-(3-Cyclopentanesulfonyl-phenyl)-butoxy]-hexylamino}-1-hydroxyethyl)-2-hydroxymethyl-phenol
- *N*-1-Adamantanyl-2-{3-[(2R)-2-({(2R)-2-hydroxy-2-[4-hydroxy-3-(hydroxymethyl)phenyl]ethyl}amino)propyl]phenyl}acetamid
- (1R)-5-{2-[6-(2,2-Difluor-2-phenyl-ethoxy)-hexylamino]-1-hydroxy-ethyl}-8-hydroxy-1H-chinolin-2-on
- (R,S)-4-(2-{[6-(2,2-Difluor-4-phenylbutoxy)hexyl]amino}-1-hydroxy-ethyl)-2-(hydroxymethyl)phenol
- (R,S)-4-(2-{[6-(2,2-Difluor-2-phenylethoxy)hexyl]amino}-1-hydroxy-ethyl)-2-(hydroxymethyl)phenol
- (R,S)-4-(2-{[4,4-Difluor-6-(4-phenylbutoxy)hexyl]amino}-1-hydroxy-ethyl)-2-(hydroxymethyl)phenol
- (R,S)-4-(2-{[6-(4,4-Difluor-4-phenylbutoxy)hexyl]amino}-1-hydroxy-ethyl)-2-(hydroxymethyl)phenol
- (R,S)-5-(2-{[6-(2,2-Difluor-2-phenylethoxy)hexyl]amino}-1-hydroxy-ethyl)-8-hydroxychinolin-2(1H)-on
- (R,S)-[2-({6-[2,2-Difluor-2-(3-methylphenyl)ethoxy]hexyl}amino)-1-hydroxyethyl]-2-(hydroxymethyl)phenol
- 4-(1R)-2-{[6-(2,2-Difluor-2-phenylethoxy)hexyl]amino}-1-hydroxyethyl)-2-(hydroxymethyl)phenol
- (R,S)-2-(Hydroxymethyl)-4-(1-hydroxy-2-{[4,4,5I5-tetrafluor-6-(3-phenylpropoxy)-hexyl]amino}ethyl)phenol
- (R,S)-[5-(2-{[6-(2,2-Difluor-2-phenylethoxy)hexyl]amino}-1-hydroxy-ethyl)-2-hydroxyphenyl]formamid
- (R,S)-4-[2-({6-[2-(3-Bromophenyl)-2,2-difluoroethoxy]hexyl}amino)-1-hydroxyethyl]-2-(hydroxymethyl)phenol
- (R, S)-N-[3-(1,1-Difluor-2-{[6-({2-hydroxy-2-[4-hydroxy-3-(hydroxymethyl)phenyl]-ethyl}amino)hexyl]oxy}ethyl)phenyl]-harnstoff
- 3-[3-(1,1-Difluor-2-{[6-({2-hydroxy-2-[4-hydroxy-3-(hydroxymethyl)phenyl]ethyl}-amino)hexyl]oxy}ethyl)phenyl]imidazolidin-2,4-dion
- (R,S)-4-[2-({6-[2,2-Difluor-2-(3-methoxyphenyl)ethoxy]hexyl}amino)-1-hydroxyethyl]-2-(hydroxymethyl)phenol
- 5-((1R)-2-{[6-(2,2-Difluor-2-phenylethoxy)hexyl]amino}-1-hydroxyethyl)-8-hydroxychinolin-2(1H)-on
- 4-((1R)-2-{[4,4-Difluor-6-(4-phenylbutoxy)hexyl]amino}-1-hydroxy-ethyl)-2-(hydroxymethyl)phenol
- (R,S)-4-(2-{[6-(3,3-Difluor-3-phenylpropoxy)hexyl]amino}-1-hydroxy-ethyl)-2-(hydroxymethyl)phenol
- (R,S)-(2-{[6-(2,2-Difluor-2-phenylethoxy)-4,4-difluorohexyl]amino}-1-hydroxyethyl)-2-(hydroxymethyl)phenol
- (R,S)-4-(2-{[6-(2,2-Difluor-3-phenylpropoxy)hexyl]amino}-1-hydroxyethyl)-2-(hydroxymethyl)phenol
- 3-[2-(3-Chlor-phenyl)-ethoxy]-N-(2-diethylamino-ethyl)-N-{2-[2-(4-hydroxy-2-oxo-2,3-dihydro-benzothiazol-7-yl)-ethylamino]-ethyl}-propionamid
- N-(2-Diethylamino-ethyl)-N-{2-[2-(4-hydroxy-2-oxo-2,3-dihydro-benzothiazol-7-yl)-ethylamino]-ethyl}-3-(2-naphthalen-1-yl-ethoxy)-propionamid
- 7-[2-(2-{3-[2-(2-Chlor-phenyl)-ethylamino]-propylsulfanyl}-ethylamino)-1-hydroxyethyl]-4-hydroxy-3H-benzothiazol-2-on
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus: Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Aclidiniumsalze, bevorzugt das Bromidsalz, Glycopyrroniumsalzen, bevorzugt das Bromidsalz, Trospiumsalzen, bevorzugt das Chloridsalz, Tolterodin, (3R)-1-Phenethyl-3-(9H-xanthen-9-carbonyloxy)-1-azoniabicyclo[2.2.2]octan-Salze. In den vorstehend genannten Salzen stellen die Kationen die pharmakologisch aktiven Bestandteile dar. Als Anionen X⁻können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt. Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus: Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, Tipredane und
- Pregna-1,4-diene-3,20-dione, 6-fluoro-11-hydroxy-16, 17-[(1-methylethylidene) bis(oxy)]-21-[[4-[(nitrooxy)methyl]benzoyl]oxy]-, (6-alpha,ll-beta,16-alpha)- (9CI) (NCX-1024)
- 16,17-butylidenedioxy-6,9-difluoro-11-hydroxy-17-(methylthio)androst-4-en-3-one (RPR-106541),
- 6,9-Difluor-17-[(2-furanylcarbonyl)oxy]-11-hydroxy-16-methyl-3 -oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3 S-yl)ester,
- 6-alpha, 9-alpha-difluoro-11-beta-hydroxy-16alpha-methyl-3-oxo-17alpha-(2,2,3,3-tetramethylcyclopropylcarbonyl)oxy-androsta-1,4-diene-17beta-carbonsäure cyanomethyl ester,
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), TofimilastPumafentrin, Lirimilast, Apremilast, Arofyllin, Atizoram, Oglemilast, Tetomilast, und
- 5-[(N-(2,5-dichloro-3-pyridinyl)-carboxamid]-8-methoxy-Chinolin (D-4418),
- N-(3,5-dichloro-1-oxido-4-pyridinyl)-carboxamid] -8-methoxy-2-(trifluoromethyl)-Chinolin (D-4396 (Sch-351591)),N-(3,5-dichloropyrid-4-yl)-[1-(4-fluorobenzyl)-5-hydroxy-indol-3-yl]glyoxylsäureamid (AWD-12-281 (GW-842470)), 9-[(2-fluorophenyl)methyl]-N-methyl-2-(trifluoromethyl)-9H-Purin-6-amine (NCS-613),
- 4-[(2R)-2-[3-(cyclopentyloxy)-4-methoxyphenyl]-2-phenylethyl]-Pyridine (CDP-840),
- N-[(3R)-3,4,6,7-tetrahydro-9-methyl-4-oxo-1-phenylpyrrolo[3,2,1-jk][1,4]benzodiazepin-3-yl]-4-Pyridinecarboxamide (PD-168787),
- 4-[6,7-diethoxy-2,3-bis(hydroxymethyl)-1-naphthalenyl]-1-(2-methoxyethyl)-2(1H)-Pyridinone (T-440),
- 2-[4-[6,7-diethoxy-2,3-bis(hydroxymethyl)-1-naphthalenyl]-2-pyridinyl]-4-(3-pyridinyl)-1(2H)-Phthalazinone (T-2585),
- (3-(3-cyclopenyloxy-4-methoxybenzyl)-6-ethylamino-8-isopropyl-3H-purine (V-11294A),
- beta-[3-(cyclopentyloxy)-4-methoxyphenyl]-1,3-dihydro-1,3-dioxo-2H-Isoindole-2-propanamid (CDC-801),
- Imidazo[1,5-a]pyrido[3,2-e]pyrazin-6(5H)-one, 9-ethyl-2-methoxy-7-methyl-5-propyl-(D-22888)
- 5-[3-(cyclopentyloxy)-4-methoxyphenyl]-3-[(3-methylphenyl)methyl]-, (3 S,5S)-2-Piperidinon (HT-0712),
- 4-[1-[3,4-bis(difluoromethoxy)phenyl]-2-(3-methyl-1-oxido-4-pyridinyl)ethyl]-alpha,alpha-bis(trifluoromethyl)-Benzenemethanol (L-826141)
- N-(3,5-Dichloro-1-oxo-pyridin-4-yl)-4-difluormethoxy-3-cyclopropylmethoxybenzamid
- (-)p-[(4*a*R*,10b*S**)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxyphenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als EGFR-Hemmer gelangen hierbei vorzugsweise Verbindungen zur Anwendung, die ausgewählt sind aus der Gruppe bestehend ausCetuximab, Trastuzumab, Panitumumab (= ABX-EGF), Mab ICR-62, Gefitinib, Canertinib, Erlotinib, und
- 4-[(3-Chlor-4-fluorphenyl)amino]-6-{[4-(morpholin-4-yl)-1-oxo-2-buten-1-yl]amino}-7-cyclopropylmethoxy-chinazolin,
- 4-[(3-Chlor-4-fluorphenyl)amino]-6-{[4-(N,N-diethylamino)-1-oxo-2-buten-1-yl]amino}-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-cyclopropylmethoxy-chinazolin,
- 4-[(R)-(1-Phenyl-ethyl)amino]-6-{[4-(morpholin-4-yl)-1-oxo-2-buten-1-yl]amino}-7-cyclopentyloxy-chinazolin,
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-cyclopentyloxy-chinazolin,
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin,
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin,
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin,
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin,
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin,
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin,
- 4-[(3 -Chlor-4-fluorphenyl)amino] -6- {[4-(N,N-dimethylamino)-1-oxo-2-buten-1-yl]amino}-7-[(R)-(tetrahydrofuran-2-yl)methoxy]-chinazolin,
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin,
- 4-[(3-Ethinyl-phenyl)amino]-6,7-bis-(2-methoxy-ethoxy)-chinazolin,
- 4-[(3-Chlor-4-fluorphenyl)amino]-7-[3-(morpholin-4-yl)-propyloxy]-6-[(vinylcarbonyl)amino]-chinazolin,
- 4-[(R)-(l-Phenyl-ethyl)amino]-6-(4-hydroxy-phenyl)-7H-pyrrolo[2,3-d]pyrimidin,
- 3-Cyano-4-[(3-chlor-4-fluorphenyl)amino]-6-{[4-(N,N-dimethylamino)-1-oxo-2-buten-1-yl]amino}-7-ethoxy-chinolin,
- 4-{[3-Chlor-4-(3-fluor-benzyloxy)-phenyl]amino}-6-(5-{[(2-methansulfonyl-ethyl)amino]methyl}-furan-2-yl)chinazolin,
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin,
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin,
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin,
- 4-[(3-Ethinyl-phenyl)amino]-6-{[4-(5,5-dimethyl-2-oxo-morpholin-4-yl)-1-oxo-2-buten-1-yl]amino}-chinazolin,
- 4-[(3-Chlor-4-fluor-phenyl)amino]-6-[2-(2,2-dimethyl-6-oxo-morpholin-4-yl)-ethoxy]-7-methoxy-chinazolin,
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin,
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin,
- 4-[(3-Chlor-4-fluor-phenyl)amino]-6-{2-[4-(2-oxo-morpholin-4-yl)-piperidin-1-yl]-ethoxy}-7-methoxy-chinazolin,
- 4-[(3-Chlor-4-fluor-phenyl)amino]-6-[1-(tert.-butyloxycarbonyl)-piperidin-4-yloxy]-7-methoxy-chinazolin,
- 4-[(3-Chlor-4-fluor-phenyl)amino] -6-(trans-4-amino-cyclohexan-1 -yloxy)-7-methoxy-chinazolin,
- 4-[(3-Chlor-4-fluor-phenyl)amino]-6-(trans-4-methansulfonylamino-cyclohexan-1-yloxy)-7-methoxy-chinazolin,
- 4-[(3-Chlor-4-fluor-phenyl)amino]-6-(tetrahydropyran-3-yloxy)-7-methoxy-chinazolin,
- 4-[(3-Chlor-4-fluor-phenyl)amino]-6-(1-methyl-piperidin-4-yloxy)-7-methoxy-chinazolin,
- 4-[(3-Chlor-4-fluor-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(methoxymethyl)carbonyl]-piperidin-4-yloxy}-7-methoxy-chinazolin,
- 4-[(3-Chlor-4-fluor-phenyl)amino]-6-(piperidin-3-yloxy)-7-methoxy-chinazolin,
- 4-[(3-Chlor-4-fluor-phenyl)amino]-6-[1-(2-acetylamino-ethyl)-piperidin-4-yloxy]-7-methoxy-chinazolin,
- 4-[(3-Chlor-4-fluor-phenyl)amino]-6-(tetrahydropyran-4-yloxy)-7-ethoxy-chinazolin,
- 4-[(3-Chlor-4-fluor-phenyl)amino]-6-((S)-tetrahydrofuran-3-yloxy)-7-hydroxy-chinazolin,
- 4-[(3-Chlor-4-fluor-phenyl)amino]-6-(tetrahydropyran-4-yloxy)-7-(2-methoxy-ethoxy)-chinazolin,
- 4-[(3-Chlor-4-fluor-phenyl)amino]-6-{trans-4-[(dimethylamino)sulfonylamino]-cyclohexan-1-yloxy}-7-methoxy-chinazolin,
- 4-[(3-Chlor-4-fluor-phenyl)amino]-6-{trans-4-[(morpholin-4-yl)carbonylamino]-cyclohexan-1-yloxy}-7-methoxy-chinazolin,
- 4-[(3-Chlor-4-fluor-phenyl)amino]-6-{trans-4-[(morpholin-4-yl)sulfonylamino]-cyclohexan-1-yloxy}-7-methoxy-chinazolin,
- 4-[(3-Chlor-4-fluor-phenyl)amino]-6-(tetrahydropyran-4-yloxy)-7-(2-acetylamino-ethoxy)-chinazolin,
- 4-[(3-Chlor-4-fluor-phenyl)amino]-6-(tetrahydropyran-4-yloxy)-7-(2-methansulfonylamino-ethoxy)-chinazolin,
- 4-[(3-Chlor-4-fluor-phenyl)amino]-6-{1-[(piperidin-1-yl)carbonyl]-piperidin-4-yloxy}-7-methoxy-chinazolin,
- 4-[(3-Chlor-4-fluor-phenyl)amino]-6-(1-aminocarbonylmethyl-piperidin-4-yloxy)-7-methoxy-chinazolin,
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin,
- 4-[(3-Chlor-4-fluor-phenyl)amino]-6-(trans-4-ethansulfonylamino-cyclohexan-1-yloxy)-7-methoxy-chinazolin,
- 4-[(3-Chlor-4-fluor-phenyl)amino]-6-(1-methansulfonyl-piperidin-4-yloxy)-7-ethoxy-chinazolin,
- 4-[(3-Chlor-4-fluor-phenyl)amino]-6-(1-methansulfonyl-piperidin-4-yloxy)-7-(2-methoxy-ethoxy)-chinazolin,
- 4-[(3-Chlor-4-fluor-phenyl)amino]-6-[1-(2-methoxy-acetyl)-piperidin-4-yloxy]-7-(2-methoxy-ethoxy)-chinazolin,
- 4-[(3-Chlor-4-fluor-phenyl)amino]-6-(cis-4-acetylamino-cyclohexan-1-yloxy)-7-methoxy-chinazolin,
- 4-[(3-Ethinyl-phenyl)amino]-6-[1-(tert.-butyloxycarbonyl)-piperidin-4-yloxy]-7-methoxy-chinazolin,
- 4-[(3-Ethinyl-phenyl)amino]-6-(tetrahydropyran-4-yloxy]-7-methoxy-chinazolin,
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin,
- 4-[(3-Chlor-4-fluor-phenyl)amino]-6-{cis-4-[(morpholin-4-yl)carbonylamino]-cyclohexan-1-yloxy}-7-methoxy-chinazolin,
- 4-[(3-Chlor-4-fluor-phenyl)amino]-6-{1-[2-(2-oxopyrrolidin-1-yl)ethyl]-piperidin-4-yloxy}-7-methoxy-chinazolin,
- 4-[(3-Chlor-4-fluor-phenyl)amino]-6-{1-[(morpholin-4-yl)carbonyl]-piperidin-4-yloxy}-7-(2-methoxy-ethoxy)-chinazolin,
- 4-[(3-Ethinyl-phenyl)amino]-6-(1-acetyl-piperidin-4-yloxy)-7-methoxy-chinazolin,
- 4-[(3-Ethinyl-phenyl)amino]-6-(1-methyl-piperidin-4-yloxy)-7-methoxy-chinazolin,
- 4-[(3-Ethinyl-phenyl)amino]-6-(l-methansulfonyl-piperidin-4-yloxy)-7-methoxy-chinazolin,
- 4-[(3-Chlor-4-fluor-phenyl)amino]-6-(1-methyl-piperidin-4-yloxy)-7(2-methoxy-ethoxy)-chinazolin,
- 4-[(3 -Chlor-4-fluor-phenyl)amino]-6-(1 -isopropyloxycarbonyl-piperidin-4-yloxy)-7-methoxy-chinazolin,
- 4-[(3-Chlor-4-fluor-phenyl)amino]-6-(cis-4-methylamino-cyclohexan-1-yloxy)-7-methoxy-chinazolin,
- 4-[(3-Chlor-4-fluor-phenyl)amino]-6-{ cis-4-[N-(2-methoxy-acetyl)-N-methyl-amino]-cyclohexan-1-yloxy}-7-methoxy-chinazolin,
- 4-[(3-Ethinyl-phenyl)amino]-6-(piperidin-4-yloxy)-7-methoxy-chinazolin,
- 4-[(3-Ethinyl-phenyl)amino]-6-[1-(2-methoxy-acetyl)-piperidin-4-yloxy]-7-methoxy-chinazolin,
- 4-[(3-Ethinyl-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(cis-2,6-dimethyl-morpholin-4-yl)carbonyl] - piperidin-4-yloxy}-7-methoxy-chinazolin,
- 4-[(3-Chlor-4-fluor-phenyl)amino]-6-{1-[(2-methyl-morpholin-4-yl)carbonyl]-piperidin-4-yloxy}-7-methoxy-chinazolin,
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin,
- 4-[(3-Chlor-4-fluor-phenyl)amino]-6-{1-[(N-methyl-N-2-methoxyethyl-amino)carbonyl]-piperidin-4-yloxy}-7-methoxy-chinazolin,
- 4-[(3-Chlor-4-fluor-phenyl)amino]-6-(1-ethyl-piperidin-4-yloxy)-7-methoxy-chinazolin,
- 4-[(3-Chlor-4-fluor-phenyl)amino]-6-{1-[(2-methoxyethyl)carbonyl]-piperidin-4-yloxy}-7-methoxy-chinazolin,
- 4-[(3-Chlor-4-fluor-phenyl)amino]-6-{-[(3-methoxypropyl-amino)-carbonyl]-piperidin-4-yloxy}-7-methoxy-chinazolin,
- 4-[(3-Chlor-4-fluor-phenyl)amino]-6-[cis-4-(N-methansulfonyl-N-methyl-amino)-cyclohexan-1-yloxy]-7-methoxy-chinazolin,
- 4-[(3-Chlor-4-fluor-phenyl)amino]-6-[cis-4-(N-acetyl-N-methyl-amino)-cyclohexan-1-yloxy]-7-methoxy-chinazolin,
- 4-[(3-Chlor-4-fluor-phenyl)amino]-6-(trans-4-methylamino-cyclohexan-1-yloxy)-7-methoxy-chinazolin,
- 4-[(3-Chlor-4-fluor-phenyl)amino]-6-[trans-4-(N-methansulfonyl-N-methyl-amino)-cyclohexan-1-yloxy]-7-methoxy-chinazolin,
- 4-[(3-Chlor-4-fluor-phenyl)amino]-6-(trans-4-dimethylamino-cyclohexan-1-yloxy)-7-methoxy-chinazolin,
- 4-[(3-Chlor-4-fluor-phenyl)amino]-6-(trans-4-{N-[(morpholin-4-yl)carbonyl]-N-methylamino}-cyclohexan-1-yloxy)-7-methoxy-chinazolin,
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin,
- 4-[(3-Chlor-4-fluor-phenyl)amino]-6-(1-methansulfonyl-piperidin-4-yloxy)-7-methoxy-chinazolin,
- 4-[(3-Chlor-4-fluor-phenyl)amino]-6-(1-cyano-piperidin-4-yloxy)-7-methoxy-chinazolin,
- 3-Cyano-4-[(3-chlor-4-fluorphenyl)amino]-6-{[4-(N,N-dimethylamino)-1-oxo-2-buten-1-yl]amino}-7-ethoxy-chinolin ;
- [4-[(3-chloro-4-fluoro-phenyl)amino]-6-{[4-(homomorpholin-4-yl)-1-oxo-2-buten-1-yl]amino}-7-[(S)-(tetrahydrofuran-3-yl)oxy]-quinazoline,
- 4-[(3-Chlor-4-fluor-phenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin,
- 4-[(3-Chlor-4-fluor-phenyl)amino]-7-[2-((S)-6-methyl-2-oxo-morpholin-4-yl)-ethoxy]-6-[(vinylcarbonyl)amino]-chinazolin,
- 4-[(3-Chlor-4-fluor-phenyl)amino]-7-[4-((R)-6-methyl-2-oxo-morpholin-4-yl)-butyloxy]-6-[(vinylcarbonyl)amino]-chinazolin,
- 4-[(3-Chlor-4-fluor-phenyl)amino]-7-[4-((S)-6-methyl-2-oxo-morpholin-4-yl)-butyloxy]-6-[(vinylcarbonyl)amino]-chinazolin, und
- 4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin,
gegebenenfalls in Form ihrer Racemate, Enantiomere oder Diastereomere, gegebenenfalls in Form ihrer pharmakologisch verträglichen Säureadditionssalze, ihrer Solvate und/oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als EGFR-Hemmer gelangen hierbei vorzugsweise Verbindungen zur Anwendung, die ausgewählt sind aus der Gruppe bestehend aus
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Ethinyl-phenyl)amino]-6,7-bis-(2-methoxy-ethoxy)-chinazolin,
- 4-[(3-Chlor-4-fluorphenyl)amino]-7-[3-(morpholin-4-yl)-propyloxy]-6-[(vinylcarbonyl)amino]-chinazolin,
- 3-Cyano-4-[(3-chlor-4-fluorphenyl)amino]-6-{[4-(N,N-dimethylamino)-1-oxo-2-buten-1-yl]amino}-7-ethoxy-chinolin,
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin,
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin,
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin,
- 4-[(3-Ethinyl-phenyl)amino]-6-{[4-(5,5-dimethyl-2-oxo-morpholin-4-yl)-1-oxo-2-buten-1-yl]amino}-chinazolin,
- 4-[(3-Chlor-4-fluor-phenyl)amino]-6-[2-(2,2-dimethyl-6-oxo-morpholin-4-yl)-ethoxy]-7-methoxy-chinazolin,
- 4- [(3 -Chlor-4-fluor-phenyl)amino] -6- [2-(2,2-dimethyl-6-oxo-morpholin-4-yl)-ethoxy] -7-[(R)-(tetrahydrofuran-2-yl)methoxy]-chinazolin,
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin,
- 4-[(3-Chlor-4-fluor-phenyl)amino]-6-{2-[4-(2-oxo-morpholin-4-yl)-piperidin-1-yl]-ethoxy}-7-methoxy-chinazolin,
- 4-[(3-Chlor-4-fluor-phenyl)amino]-6-[1-(tert.-butyloxycarbonyl)-piperidin-4-yloxy]-7-methoxy-chinazolin,
- 4-[(3-Chlor-4-fluor-phenyl)amino]-6-(trans-4-amino-cyclohexan-1-yloxy)-7-methoxy-chinazolin,
- 4-[(3-Chlor-4-fluor-phenyl)amino]-6-(trans-4-methansulfonylamino-cyclohexan-1-yloxy)-7-methoxy-chinazolin,
- 4-[(3-Chlor-4-fluor-phenyl)amino]-6-(tetrahydropyran-3-yloxy)-7-methoxy-chinazolin,
- 4-[(3-Chlor-4-fluor-phenyl)amino]-6-(1-methyl-piperidin-4-yloxy)-7-methoxy-chinazolin,
- 4-[(3-Chlor-4-fluor-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(methoxymethyl)carbonyl]-piperidin-4-yloxy}-7-methoxy-chinazolin,
- 4-[(3-Chlor-4-fluor-phenyl)amino]-6-(piperidin-3-yloxy)-7-methoxy-chinazolin,
- 4-[(3-Chlor-4-fluor-phenyl)amino]-6-[1-(2-acetylamino-ethyl)-piperidin-4-yloxy]-7-methoxy-chinazolin,
- 4-[(3-Chlor-4-fluor-phenyl)amino]-6-(tetrahydropyran-4-yloxy)-7-ethoxy-chinazolin,
- 4-[(3-Chlor-4-fluor-phenyl)amino]-6-((S)-tetrahydrofuran-3-yloxy)-7-hydroxy-chinazolin,
- 4-[(3-Chlor-4-fluor-phenyl)amino]-6-(tetrahydropyran-4-yloxy)-7-(2-methoxy-ethoxy)-chinazolin,
- 4-[(3-Chlor-4-fluor-phenyl)amino]-6-{trans-4-[(dimethylamino)sulfonylamino]-cyclohexan-1-yloxy}-7-methoxy-chinazolin,
- 4-[(3-Chlor-4-fluor-phenyl)amino]-6-{trans-4-[(morpholin-4-yl)carbonylamino]-cyclohexan-1-yloxy}-7-methoxy-chinazolin,
- 4-[(3-Chlor-4-fluor-phenyl)amino]-6-{trans-4-[(morpholin-4-yl)sulfonylamino]-cyclohexan-1-yloxy}-7-methoxy-chinazolin,
- 4-[(3-Chlor-4-fluor-phenyl)amino]-6-(tetrahydropyran-4-yloxy)-7-(2-acetylamino-ethoxy)-chinazolin,
- 4-[(3-Chlor-4-fluor-phenyl)amino]-6-(tetrahydropyran-4-yloxy)-7-(2-methansulfonylamino-ethoxy)-chinazolin,
- 4-[(3-Chlor-4-fluor-phenyl)amino]-6-{1-[(piperidin-1-yl)carbonyl]-piperidin-4-yloxy}-7-methoxy-chinazolin,
- 4-[(3-Chlor-4-fluor-phenyl)amino]-6-(1-aminocarbonylmethyl-piperidin-4-yloxy)-7-methoxy-chinazolin,
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin,
- 4-[(3-Chlor-4-fluor-phenyl)amino]-6-(trans-4-ethansulfonylamino-cyclohexan-1-yloxy)-7-methoxy-chinazolin,
- 4-[(3-Chlor-4-fluor-phenyl)amino]-6-(1-methansulfonyl-piperidin-4-yloxy)-7-ethoxy-chinazolin,
- 4-[(3-Chlor-4-fluor-phenyl)amino]-6-(1-methansulfonyl-piperidin-4-yloxy)-7-(2-methoxy-ethoxy)-chinazolin,
- 4-[(3-Chlor-4-fluor-phenyl)amino]-6-[1-(2-methoxy-acetyl)-piperidin-4-yloxy]-7-(2-methoxy-ethoxy)-chinazolin,
- 4-[(3-Chlor-4-fluor-phenyl)amino]-6-(cis-4-acetylamino-cyclohexan-1-yloxy)-7-methoxy-chinazolin,
- 4-[(3-Ethinyl-phenyl)amino]-6-[1-(tert.-butyloxycarbonyl)-piperidin-4-yloxy]-7-methoxy-chinazolin,
- 4-[(3-Ethinyl-phenyl)amino]-6-(tetrahydropyran-4-yloxy]-7-methoxy-chinazolin,
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin,
- 4-[(3-Chlor-4-fluor-phenyl)amino]-6-{cis-4-[(morpholin-4-yl)carbonylamino]-cyclohexan-1-yloxy}-7-methoxy-chinazolin,
- 4-[(3-Chlor-4-fluor-phenyl)amino]-6-{1-[2-(2-oxopyrrolidin-1-yl)ethyl]-piperidin-4-yloxy}-7-methoxy-chinazolin,
- 4-[(3-Chlor-4-fluor-phenyl)amino]-6-{1-[(morpholin-4-yl)carbonyl]-piperidin-4-yloxy} - 7-(2-methoxy-ethoxy)-chinazolin,
- 4-[(3-Ethinyl-phenyl)amino]-6-(1-acetyl-piperidin-4-yloxy)-7-methoxy-chinazolin,
- 4-[(3-Ethinyl-phenyl)amino]-6-(1-methyl-piperidin-4-yloxy)-7-methoxy-chinazolin,
- 4-[(3-Ethinyl-phenyl)amino]-6-(1-methansulfonyl-piperidin-4-yloxy)-7-methoxy-chinazolin,
- 4-[(3-Chlor-4-fluor-phenyl)amino]-6-(1-methyl-piperidin-4-yloxy)-7(2-methoxy-ethoxy)-chinazolin,
- 4-[(3-Chlor-4-fluor-phenyl)amino]-6-(1-isopropyloxycarbonyl-piperidin-4-yloxy)-7-methoxy-chinazolin,
- 4-[(3-Chlor-4-fluor-phenyl)amino]-6-(cis-4-methylamino-cyclohexan-1-yloxy)-7-methoxy-chinazolin,
- 4-[(3-Chlor-4-fluor-phenyl)amino]-6-{cis-4-[N-(2-methoxy-acetyl)-N-methyl-amino] - cyclohexan-1-yloxy}-7-methoxy-chinazolin,
- 4-[(3-Ethinyl-phenyl)amino]-6-(piperidin-4-yloxy)-7-methoxy-chinazolin,
- 4-[(3-Ethinyl-phenyl)amino]-6-[1-(2-methoxy-acetyl)-piperidin-4-yloxy]-7-methoxy-chinazolin,
- 4-[(3-Ethinyl-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(cis-2,6-dimethyl-morpholin-4-yl)carbonyl] - piperidin-4-yloxy}-7-methoxy-chinazolin,
- 4-[(3-Chlor-4-fluor-phenyl)amino]-6-{1-[(2-methyl-morpholin-4-yl)carbonyl]-piperidin-4-yloxy}-7-methoxy-chinazolin,
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin,
- 4-[(3-Chlor-4-fluor-phenyl)amino]-6-{1-[(N-methyl-N-2-methoxyethyl-amino)carbonyl]-piperidin-4-yloxy}-7-methoxy-chinazolin,
- 4-[(3-Chlor-4-fluor-phenyl)amino]-6-(1-ethyl-piperidin-4-yloxy)-7-methoxy-chinazolin,
- 4-[(3-Chlor-4-fluor-phenyl)amino]-6-{1-[(2-methoxyethyl)carbonyl]-piperidin-4-yloxy}-7-methoxy-chinazolin,
- 4-[(3-Chlor-4-fluor-phenyl)amino]-6-{1-[(3-methoxypropyl-amino)-carbonyl]-piperidin-4-yloxy}-7-methoxy-chinazolin,
- 4-[(3-Chlor-4-fluor-phenyl)amino]-6-[cis-4-(N-methansulfonyl-N-methyl-amino)-cyclohexan-1-yloxy]-7-methoxy-chinazolin,
- 4-[(3-Chlor-4-fluor-phenyl)amino]-6-[cis-4-(N-acetyl-N-methyl-amino)-cyclohexan-1-yloxy]-7-methoxy-chinazolin,
- 4-[(3-Chlor-4-fluor-phenyl)amino]-6-(trans-4-methylamino-cyclohexan-1-yloxy)-7-methoxy-chinazolin,
- 4-[(3-Chlor-4-fluor-phenyl)amino]-6-[trans-4-(N-methansulfonyl-N-methyl-amino)-cyclohexan-1-yloxy]-7-methoxy-chinazolin,
- 4-[(3-Chlor-4-fluor-phenyl)amino]-6-(trans-4-dimethylamino-cyclohexan-1-yloxy)-7-methoxy-chinazolin,
- 4-[(3-Chlor-4-fluor-phenyl)amino]-6-(trans-4-{N-[(morpholin-4-yl)carbonyl]-N-methylamino}-cyclohexan-1-yloxy)-7-methoxy-chinazolin,
- 4-[(3-Chlor-4-fluor-phenyl)amino]-6- [2-(2,2-dimethyl-6-oxo-morpholin-4-yl)-ethoxy] -7-[(S)-(tetrahydrofuran-2-yl)methoxy]-chinazolin,
- 4-[(3-Chlor-4-fluor-phenyl)amino]-6-(1-methansulfonyl-piperidin-4-yloxy)-7-methoxy-chinazolin,
- 4-[(3-Chlor-4-fluor-phenyl)amino]-6-(1-cyano-piperidin-4-yloxy)-7-methoxy-chinazolin,
- 3-Cyano-4-[(3-chlor-4-fluorphenyl)amino]-6-{[4-(N,N-dimethylamino)-1-oxo-2-buten-1-yl]amino}-7-ethoxy-chinolin
- [4-[(3-chloro-4-fluoro-phenyl)amino]-6-{[4-(homomorpholin-4-yl)-1-oxo-2-buten-1-yl]amino}-7-[(S)-(tetrahydrofuran-3-yl)oxy]-chinazoline
- 4-[(3-Chlor-4-fluor-phenyl)amino]-7-(2-{4-[(S)-(2-oxo-tetrahydrofuran-5-yl)carbonyl] - piperazin-1-yl}-ethoxy)-6-[(vinylcarbonyl)amino]-chinazolin
- 4-[(3-Chlor-4-fluor-phenyl)amino]-7-[2-((S)-6-methyl-2-oxo-morpholin-4-yl)-ethoxy]-6-[(vinylcarbonyl)amino]-chinazolin
- 4-[(3-Chlor-4-fluor-phenyl)amino]-7-[4-((R)-6-methyl-2-oxo-morpholin-4-yl)-butyloxy] - 6-[(vinylcarbonyl)amino]-chinazolin
- 4- [(3 -Chlor-4-fluor-phenyl)amino] -7- [4-((S)-6-methyl-2-oxo-morpholin-4-yl)-butyloxy] - 6-[(vinylcarbonyl)amino]-chinazolin
- 4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin
Cetuximab, Trastuzumab, ABX-EGF, Mab ICR-62, Gefitinib, Canertinib und Erlotinib, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Rezeptor Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als PAF-Antagonisten gelangen hierbei vorzugsweise Verbindungen zur Anwendung, die ausgewählt sind aus der Gruppe bestehend aus Lexipafant und 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin
- 6-(2-Chlorphenyl)-8,9-dihydro-1-methyl-8-[(4-morpholinyl)carbonyl]-4H,7H-cyclopenta-[4,5]thieno-[3,2-f][1,2,4]triazolo[4,3-a][1,4]diazepin,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTB4-Rezeptor Antagonisten gelangen hierbei vorzugsweise Verbindungen zur Anwendung wie z.B. Amebulant (=[[4-[[3-[[4-[1-(4-hydroxyphenyl)-1-methylethyl]phenoxy]methyl]phenyl]methoxy]phenyl]iminomethyl]-Carbaminsäure-ethyl ester), gegebenenfalls in Form ihrer Racemate, Enantiomere, Diastereomere und gegebenenfalls in Form ihrer pharmakologisch verträglichen Säureadditionssalze, Solvate, Prodrugs oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Rezeptor Antagonisten gelangen hierbei vorzugsweise Verbindungen zur Anwendung, die ausgewählt sind aus der Gruppe bestehend aus Montelukast, Pranlukast, Zafirlukast, und (E)-8-[2-[4-[4-(4-Fluorophenyl)butoxy]phenyl]ethenyl]-2-(1H-tetrazol-5-yl)-4H-1-benzopyran-4-one (MEN-91507)
- 4-[6-Acetyl-3-[3-(4-acetyl-3-hydroxy-2-propylphenylthio)propoxy]-2-propylphenoxy] - buttersäure (MN-001)
- 1-(((R)-(3-(2-(6,7-Difluor-2-chinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yI)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Rezeptor Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als Histamin H1 Rezeptor Antagonisten gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Olopatadine, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Histamin H4 Rezeptor Antagonisten gelangen hierbei vorzugsweise Verbindungen zur Anwendung wie z.B. (5-chloro-1H-indol-2-yl)(4-methyl-1-piperazinyl)-Methanone (JNJ-7777120), gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat

Als Inhibitoren von Nicht-Rezeptor Tyrosine Kinasen wie zum Beispiel LYN, LCK, SYK, ZAP-70, FYN, BTK oder ITK gelangen hierbei vorzugsweise Verbindungen zur Anwendung, die ausgewählt sind aus der Gruppe bestehend aus
- 2-[(2-aminoethyl)amino]-4-[(3-bromophenyl)amino]-5-Pyrimidinecarboxamid;
- 2-[[7-(3,4-dimethoxyphenyl)imidazo[1,2-c]pyrimidin-5-yl]amino]-3-Pyridinecarboxamid;
- 6-[[5-fluoro-2-[3,4,5-trimethoxyphenyl)amino]-4-pyrimidinyl]amino]-2,2-dimethyl-2H-Pyrido[3,2-b]-1,4-oxazin-3(4H)-on;
- N-[3-bromo-7-(4-methoxyphenyl)-1,6-naphthyridin-5-yl]-1,3-Propanediamin
- 7-(4-methoxyphenyl)-N-methyl-1,6-Naphthyridin-5-amin;
- N-[7-(4-methoxyphenyl)-1,6-naphthyridin-5-yl]-1,3-Propanediamin;
- N-[7-(2-thienyl)-1,6-naphthyridin-5-yl-1,3-Propanediamin;
- N-[7-[4-(dimethylamino)phenyl]-1,6-naphthyridin-5-yl]-1,2-Ethanediamin;
- N-[7-(4-methoxyphenyl)-2-(trifluoromethyl)-1,6-naphthyridin-5-yl]-1,3-Propanediamin;
- N-[7-(4-methoxyphenyl)-3-phenyl-1,6-naphthyridin-5-yl]-1,3-Propanediamin;
- N-(7-phenyl-1,6-naphthyridin-5-yl)-1,3-Propanediamin;
- N-[7-(3-fluorophenyl)-1,6-naphthyridin-5-yl]-1,3-Propanediamin;
- N-[7-(3-chlorophenyl)-1,6-naphthyridin-5-yl]-1,3-Propanediamin;
- N-[7-[3-(trifluoromethoxy)phenyl]-1,6-naphthyridin-5yl]-1,3-Propanediamin;
- N-[7-(4-fluorophenyl)-1,6-naphthyridin-5-yl]-1,3-Propanediamin;
- N-[7-(4-fluorophenyl)-1,6-naphthyridin-5-yl]-1,3-Propanediamin;
- N-[7-(4-chlorophenyl)-1,6-naphthyridin-5-yl]-1,3-Propanediamin;
- N-[7-(4'-methyl[1,1'-biphenyl]-4-yl)-1,6-naphthyridin-1,3-Propanediamin;
- N-[7-[4-(dimethylamino)phenyl]-1,6-naphthyridin-5-yl]-1,3-Propanediamin;
- N-[7-[4-(diethylamino)phenyl]-1,6-naphthyridin-5-yl]-1,3-Propanediamin;
- N-[7-[4-(4-morpholinyl)phenyl]-1,6-naphthyridin-5-yl]-1,3-Propanediamin;
- N-[7-[4-[[2-(dimethylamino)ethyl]methylamino]phenyl]-1,6-naphthyridin-5-yl]-1,3-Propanediamin;
- N-[7-(4-bromophenyl)-1,6-naphthyridin-5-yl]-1,3-Propanediamin;
- N-[7-(4-methylphenyl)-1,6-naphthyridin-5-yl]-1,3-Propanediamin;
- N-[7-[4-(methylthio)phenyl]-1,6-naphthyridin-5-yl]-1,3-Propanediamin;
- N-[7-[4-(1-methylethyl)phenyl]-1,6-naphthyridin-5-yl]-1,3-Propanediamin;
- 7-[4-(dimethylamino)phenyl]-N-methyl-1,6-Naphthyridin-5-amin;
- 7- [4-(dimethylamino)phenyl] -N,N-dimethyl-1, 6-Naphthyridin-5 -amin;
- N-[7-[4-(dimethylamino)phenyl]-1,6-naphthyridin-5-yl]-1,4-Butanediamin;
- N-[7-[4-(dimethylamino)phenyl]-1,6-naphthyridin-5-yl]-1,5-Pentanediamin;
- 3-[[7-[4-(dimethylamino)phenyl]-1,6-naphthyridin-5-yl]oxy]-1-Propanol;
- 4-[5-(4-aminobutoxy)-1,6-naphthyridin-7-yl]-N,N-dimethyl-Benzenamin,
- 4-[[7-[4-(dimethylamino)phenyl]-1,6-naphthyridin-5-yl]amino]-1-Butanol;
- N-[7-[4-(dimethylamino)phenyl]-1,6-naphthyridin-5-yl]-N-methyl-1,3-Propanediamin;
- N-[7-[4-(dimethylamino)phenyl]-1,6-naphthyridin-5-yl]-N'-methyl-1,3-Propanediamin;
- N-[7-[4-(dimethylamino)phenyl]-1,6-naphthyridin-5-yl]-N,N'-dimethyl-1,3-Propanediamin;
- 1-amino-3-[[7-[4-(dimethylamino)phenyl]-1,6-naphthyridin-5-yl]amino]-2-Propanol;
- N-[7-[4-(dimethylamino)phenyl]-1,6-naphthyridin-5-yl]-2,2-dimethyl-1,3-Propanediamin;
- 7-[4-(dimethylamino)phenyl]-N-(3-pyridinylmethyl)-1,6-Naphthyridin-5-amin;
- N-[(2-aminophenyl)methyl]-7-[4-(dimethylamino)phenyl]-1,6-Naphthyridin-5-amin;
- N-[7-[6-(dimethylamino)[1,1'-biphenyl]-3-yl]-1,6-naphthyridin-5-yl]-1,3-Propanediamin;
- N-[7-[3-chloro-4-(diethylamino)phenyl]-1,6-naphthyridin-5-yl]-1,3-Propanediamin;
- N-[7-[4-(dimethylamino)-3-methoxyphenyl]-1,6-naphthyridin-5-yl]-1,3-Propanediamin;
- N-[7-[4-(diethylamino)phenyl]-3-methyl-1,6-naphthyridin-5-yl]-1,3-Propanediamin;
- N-[7-(3'-fluoro[1,1'-biphenyl]-3-yl)-1,6-naphthyridin-5-yl]-1,2-Ethanediamin,
- N-[7-(4-methoxyphenyl)-1,6-naphthyridin-5-yl]-1,6-Naphthyridine-1,3-Propanediamin;
- N,N'-bis(3-aminopropyl)-7-(4-methoxyphenyl)-2,5-diamin;
- N-[7-(4-methoxyphenyl)-2-(phenylmethoxy)-1,6-naphthyridin-5-yl]-1,6-Naphthyridine-1,3-Propanediamin;
- N5-(3-aminopropyl)-7-(4-methoxyphenyl)-N2-(phenylmethyl)-2,5-diamin;
- N-[7-(2-naphthalenyl)-1,6-naphthyridin-5-yl]-1,3-Propanediamin;
- N-[7-(2'-fluoro[1,1'-biphenyl]-4-yl)-1,6-naphthyridin-5-yl]-1,3-Propanediamin;
- N-[7-(3,4,5-trimethoxyphenyl)-1,6-naphthyridin-5-yl]-1,3-Propanediamin;
- N-[7-(3,4-dimethylphenyl)-1,6-naphthyridin-5-yl]-1,3-Propanediamin;
- 1-amino-3-[[7-(2-naphthalenyl)-1,6-naphthyridin-5-yl]amino]-2-Propanol;
- 1-amino-3-[[7-(2'-fluoro[1,1'-biphenyl]-4-yl)-1,6-naphthyridin-5-yl]amino]-2-Propanol;
- 1-amino-3-[[7-(4'-methoxy[1,1'-biphenyl]-4-yl)-1,6-naphthyridin-5-yl]amino]-2-Propanol;
- 1-amino-3-[[7-(3,4,5-trimethoxyphenyl)-1,6-naphthyridin-5-yl]amino]-2-Propanol;
- 1-amino-3-[[7-(4-bromophenyl)-1,6-naphthyridin-5-yl]amino]-2-Propanol;
- N-[7-(4'-methoxy[1,1'-biphenyl]-4-yl)-1,6-naphthyridin-5-yl]-2,2-dimethyl-1,3-Propanediamin;
- 1-[[7-[4-(dimethylamino)phenyl]-1,6-naphthyridin-5-yl]amino]-2-Propanol;
- 2-[[2-[[7-[4-(dimethylamino)phenyl]-1,6-naphthyridin-5-yl]amino]ethyl]thio]-Ethanol;
- 7-[4-(dimethylamino)phenyl]-N-(3-methyl-5-isoxazolyl)-1,6-Naphthyridin-5-amin;
- 7-[4-(dimethylamino)phenyl]-N-4-pyrimidinyl-1,6-Naphthyridin-5-amin;
- N-[7-[4-(dimethylamino)phenyl]-1,6-naphthyridin-5-yl]-1,3-Cyclohexanediamin;
- N,N-dimethyl-4-[5-(1-piperazinyl)-1,6-naphthyridin-7-yl]-Benzenamin;
- 4-[5-(2-methoxyethoxy)-1,6-naphthyridin-7-yl]-N,N-dimethyl-Benzenamin;
- 1-[7-[4-(dimethylamino)phenyl]-1,6-naphthyridin-5-yl]-4-Piperidinol;
- 1-[7-[4-(dimethylamino)phenyl]-1,6-naphthyridin-5-yl]-3-Pyrrolidinol;
- 7-[4-(dimethylamino)phenyl]-N-(2-furanylmethyl)-1,6-Naphthyridin-5-amin;
- 7-[4-(dimethylamino)phenyl]-N-[3-(1H-imidazol-1-yl)propyl]-1,6-Naphthyridin-5-amin;
- 1-[7-[4-(dimethylamino)phenyl]-1,6-naphthyridin-5-yl]-4-Piperidinecarboxamid;
- 1-[3-[[7-[4-(dimethylamino)phenyl]-1,6-naphthyridin-5-yl]amino]propyl]-2-Pyrrolidinon;
- N-[3'-[5-[(3-aminopropyl)amino]-1,6-naphthyridin-7-yl][1,1'-biphenyl]-3-yl]-Acetamid;
- N-[7-(4'-fluoro[1,1'-biphenyl] -4-yl)-1,6-naphthyridin-5-yl]-1,3-Propanediamin;
- N-[4'-[5-[(3-aminopropyl)amino]-1,6-naphthyridin-7-yl][1,1'-biphenyl]-3-yl]-Acetamid;
- N-[7-[4-(1,3-benzodioxol-5-yl)phenyl]-1,6-naphthyridin-5-yl]-1,3-Propanediamin;
- N-[7-[4-(2-thienyl)phenyl]-1,6-naphthyridin-5-yl]-1,3-Propanediamin;
- N-[7-[4-fluoro-3-(trifluoromethyl)phenyl]-1,6-naphthyridin-5-yl]-1,3-Propanediamin;
- N-[7-[4-(3-pyridinyl)phenyl]-1,6-naphthyridin-5-yl]-1,3-Propanediamin;
- N-[7-(1,3 -benzodioxol-5-yl)-1,6-naphthyridin-5-yl]-1,3-Propanediamin;
- N-[7-(6-methoxy-2-naphthalenyl)-1,6-naphthyridin-5-yl]-1,3-Propanediamin;
- 7-[4-(dimethylamino)phenyl]-N-(4-pyridinylmethyl)-1,6-Naphthyridin-5-amin;
- 3-[[7-[4-(dimethylamino)phenyl]-1,6-naphthyridin-5-yl]methylamino]-Propanenitril;
- 7-[4-(dimethylamino)phenyl]-N-[1-(phenylmethyl)-4-piperidinyl]-1,6-Naphthyridin-5-amin;
- N-[7-[4-(dimethylamino)phenyl]-1,6-naphthyridin-5-yl]-1,2-Cyclohexanediamin,
- N-[7-[4-(dimethylamino)phenyl]-1,6-naphthyridin-5-yl]-1,2-Cyclohexanediamine, (1R,2S)-rel-.
- N-[7-[4-(dimethylamino)phenyl]-1,6-naphthyridin-5-yl]-1,2-Benzenedimethanamine;
- N-[7-[4-(diethylamino)phenyl]-1,6-naphthyridin-5-yl]-1,4-Butanediamine;
- N-[7-[3',5'-bis(trifluoromethyl)[1,1'-biphenyl]-4-yl]-1,6-naphthyridin-5-yl]-,3-Propanediamine;
- N-[7-(3'-methoxy[1,1'-biphenyl]-4-yl)-1,6-naphthyridin-5-yl]-1,3-Propanediamine;
- N-[7-(3'-fluoro[1,1'-biphenyl]-4-yl)-1,6-naphthyridin-5-yl]-1,3-Propanediamine;
- 4-[[7-[4-(dimethylamino)phenyl]-1,6-naphthyridin-5-yl]oxy]-1-Butanol;
- N-[7-[4-(dimethylamino)phenyl]-1,6-naphthyridin-5-yl]-1,4-Cyclohexanediamine;
- 7-[4-(dimethylamino)phenyl]-N-(2,2,6,6-tetramethyl-4-piperidinyl)-1,6-Naphthyridin-5-amine;
- N-[7-[3-bromo-4-(dimethylamino)phenyl]-1,6-naphthyridin-5-yl]-1,3-Propanediamine;
- N-[7-(1-methyl-1H-indol-5-yl)-1,6-naphthyridin-5-yl]-1,3-Propanediamine;
- N-[7-[3-(trifluoromethyl)phenyl]-1,6-naphthyridin-5-yl]-1,3-Propanediamine;
- N-[7-[4-(trifluoromethyl)phenyl]-1,6-naphthyridin-5-yl]-1,3-Propanediamine;
- N-[7-(3-bromo-4-methoxyphenyl)-1,6-naphthyridin-5-yl]-1,3-Propanediamine;
- N-[7-[4-[[3-(dimethylamino)propyl]methylamino]phenyl]-1,6-naphthyridin-5-yl]-1,4-Cyclohexanediamine;
- N-[7-[4-[[2-(dimethylamino)ethyl] methylamino]phenyl]-1,6-naphthyridin-5-yl]-1,4-Cyclohexanediamine;
- N-[7-[4-(dimethylamino)-3-methoxyphenyl]-1,6-naphthyridin-5-yl]-1,4-Cyclohexanediamine;
- N-[7-[4-(4-morpholinyl)phenyl]-1,6-naphthyridin-5-yl]-1,4-Cyclohexanediamine;
- N-[7-[3-bromo-4-(4-morpholinyl)phenyl]-1,6-naphthyridin-5-yl]-1,4-Cyclohexanediamine;
- 4-[[7-[4-[[2-(dimethylamino)ethyl]methylamino]phenyl]-1,6-naphthyridin-5-yl]oxy]-Cyclohexanol;
- N-[7-[3-bromo-4-(4-morpholinyl)phenyl]-1,6-naphthyridin-5-yl]-1,3-Propanediamine;
- N,N-dimethyl-4-[5-(4-methyl-1-piperazinyl)-1,6-naphthyridin-7-yl]-Benzenamine;
- 4-[[7-[4-[[3-(dimethylamino)propyl]methylamino]phenyl]-1,6-naphthyridin-5-yl]oxy]-Cyclohexanol;
- N-[7-[4-[[2-(dimethylamino)ethyl]methylamino]phenyl]-1,6-naphthyridin-5-yl]-1,4-Butanediamin;
- [3-[[5-[(3-aminopropyl)amino]-7-(4-methoxyphenyl)-1,6-naphthyridin-2-yl]amino]propyl]-Carbaminsäure-1,1-dimethylethyl ester,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat

Als MAP Kinase Inhibitoren gelangen hierbei vorzugsweise Verbindungen zur Anwendung, die ausgewählt sind aus der Gruppe bestehend aus:
- Bentamapimod (AS-602801)
- Doramapimod (BIRB-796),
- 5-Carbamoylindole (SD-169),
- 6-[(aminocarbonyl)(2,6-difluorophenyl)amino]-2-(2,4-difluorophenyl)-3-Pyridinecarboxamide (VX-702),
- alpha- [2-[[2-(3 -pyridinyl)ethyl]amino]-4-pyrimidinyl]-2-Benzothiazoleacetonitrile (AS-601245),
- 9,12-Epoxy-1H-diindolo[1,2,3-fg:3',2',1'-kl]pyrrolo[3,4-i][1,6]benzodiazocine-10-Carboxylsäure (CEP-1347),
- 4-[3-(4-chlorophenyl)-5-(1-methyl-4-piperidinyl)-1H-pyrazol-4-yl]-Pyrimidine (SC-409),
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als iNOS-Inhibitoren gelangen hierbei vorzugsweise Verbindungen zur Anwendung, die ausgewählt sind aus der Gruppe bestehend aus: S-(2-Aminoethyl)isothioharnstoff, Aminoguanidin, 2-Aminomethylpyridin, 5,6-dihydro-6-methyl-4H-1,3-Thiazin-2-amine (AMT), L-Canavanin, 2-Iminopiperidin, S-Isopropylisothioharnstoff, S-Methylisothioharnstoff, S-Ethylisothioharnstoff, S-Methyltiocitrullin, S-Ethylthiocitrullin, L-NA (N^{ω}-Nitro-L-arginin), L-NAME (N^{ω}-Nitro-L-argininmethylester), L-NMMA (N^{ω}-Monomethyl-L-arginin), L-NIO (N^{ω}-Iminoethyl-L-ornithin), L-NIL (N^{ω}-Iminoethyl-lysin), (S)-6-Acetimidoylamino-2-amino-Hexansäure (1*H*-tetrazol-5-yl)-amid (SC-51), N-[[3-(aminomethyl)phenyl]methyl]-Ethanimidamide (1400W), (S)-4-(2-Acetimidoylamino-ethylsulfanyl)-2-amino-buttersäure (GW274150), 2-[2-(4-Methoxy-pyridin-2-yl)-ethyl]-3*H-*imidazo[4,5-*b*]pyridin (BYK191023), 2-((R)-3-Amino-1-phenyl-propoxy)-4-chlor-5-fluorbenzonitril, 2-((1R,3S)-3-Amino-4-hydroxy-1-thiazol-5-yl-butylsulfanyl)-6-trifluoromethyl-nicotinonitril, 2-((1R,3S)-3-Amino-4-hydroxy-1-thiazol-5-yl-butylsulfanyl)-4-chlor-benzonitril, 2-((1R,3S)-3-Amino-4-hydroxy-1-thiazol-5-yl-butylsulfanyl)-5-chlorbenzonitril, (2S,4R)-2-Amino-4-(2-chlor-5-trifluoromethyl-phenylsulfanyl)-4-thiazol-5-yl-butan-1-ol, 2-((1R,3S)-3-Amino-4-hydroxy-1-thiazol-5-yl-butylsulfanyl)-5-chlor-nicotinonitril, 4-((S)-3-Amino-4-hydroxy-1-phenyl-butylsulfanyl)-6-methoxy-nicotinonitril, substituierte 3-Phenyl-3,4-dihydro-1-Isochinolinamin wie z.B. (1S,5S,6R)-7-Chlor-5-methyl-2-aza-bicyclo[4.1.0]hept-2-en-3-ylamin (ONO-1714), (4R,5R)-5-Ethyl-4-methyl-thiazolidin-2-ylideneamin, (4R,5R)-5-Ethyl-4-methyl-selenazolidin-2-ylideneamin, 4-Aminotetrahydrobiopterin, (E)-3-(4-Chlor-phenyl)-*N*(1-{2-oxo-2-[4-(6-trifluormethyl-pyrimidin-4-yloxy)-piperidin-1-yl]-ethylcarbamoyl}-2-pyridin-2-yl-ethyl)-acrylamid (FR260330), 3-(2,4-Difluorphenyl)-6-[2-(4-imidazol-1-ylmethyl-phenoxy)-ethoxy] -2-phenyl-pyridin (PPA250), 3-{[(Benzo[1,3]dioxol-5-ylmethyl)-carbamoyl]-methyl}-4-(2-imidazol-1-yl-pyrimidin-4-yl)-piperazin-1-carbonsäuremethylester (BBS-1), (R)-1-(2-Imidazol-1-yl-6-methyl-pyrimidin-4-yl)-pyrrolidin-2-carbonsäure (2-benzo[1,3]dioxol-5-yl-ethyl)-amid (BBS-2) und deren pharmazeutischen Salze, Prodrugs oder Solvate.

Als iNOS-Inhibitoren im Rahmen der vorliegenden Erfindung können weiterhin antisense-Oligonucleotide, insbesondere solche antisense-Oligonucleotide, die iNOS-kodierende Nukleinsäuren binden, eingesetzt werden. Z.B. werden in WO 01/52902 antisense-Oligonucleotide, insbesondere antisense-Oligonucleotide, die iNOS kodierende Nukleinsäuren binden, zur Modulierung der Expression von iNOS beschrieben.

Als MRP4-Inhibitoren gelangen hierbei vorzugsweise Verbindungen zur Anwendung, die ausgewählt sind aus der Gruppe bestehend aus *N*-Acetyl-dinitrophenyl-Cysteine, cGMP, Cholate, Diclofenac, Dehydroepiandrosterone 3-glucuronide, Dehydroepiandrosterone 3-sulphate, Dilazep, Dinitrophenyl-*S*-glutathione, Estradiol 17-beta-glucuronide, Estradiol 3,17-disulphate, Estradiol 3-glucuronide, Estradiol 3-sulphate, Estrone 3-sulphate, Flurbiprofen, Folate, N5-formyl-tetrahydrofolate, Glykocholat, Glykolithocholsäure sulfat,, Ibuprofen, Indomethacin, Indoprofen, Ketoprofen, Lithocholsäuresulfat sulphat, Methotrexat, ((*E*)-3-[[[3-[2-(7-Chloro-2-chinolinyl)ethenyl]phenyl]-[[3-dimethylamino)-3-oxopropyl]thio]methyl]thio]-propansäure), alpha-Naphthyl-beta-D-glucuronid, Nitrobenzyl mercaptopurine ribosid, Probenecid, Sildenafil, Sulfinpyrazone, Taurochenodeoxycholate, Taurocholat, Taurodeoxycholat, Taurolithocholat, Topotecan, Trequinsin, Zaprinast und Dipyridamol, gegebenenfalls in Form ihrer Racemate, Enantiomere, Diastereomere und ihrer pharmakologisch verträglichen Säure-Additionssalze und Hydrate.

Als Leukotriene Biosynthese Inhibitoren wie zum Beispiel aus der Gruppe der 5-Lipoxygenase (5-LO) Inhibitoren, cPLA2 Inhibitoren, Leukotriene A4 Hydrolase Inhibitoren oder FLAP Inhibitoren, gelangen hierbei vorzugsweise Verbindungen zur Anwendung, die ausgewählt sind aus der Gruppe bestehend aus Zileuton, Tipelukast, Licofelone, Darapladib, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Nicht-steroidale antientzündliche Agenzien (NSAIDs) gelangen hierbei vorzugsweise Verbindungen zur Anwendung, die ausgewählt sind aus der Gruppe bestehend aus Piroxicam, Diclofenac, Naproxen, Flurbiprofen, Fenoprofen, Ketoprofen, Ibuprofen, Nimesulide, Indomethacin, Sulindac, Azapropazone, Phenylbutazone, Aspirin; Meloxicam, Celecoxib, Rofecoxib, Valdecoxib, Lumarocoxib, Parecoxib, Tenoxicam und Etoricoxib, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als CRTH2 Antagonisten gelangen hierbei vorzugsweise Verbindungen zur Anwendung, die ausgewählt sind aus der Gruppe bestehend aus Ramatroban und Laropiprant, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als DP1-Rezeptor Modulatoren gelangen hierbei vorzugsweise Verbindungen zur Anwendung, die ausgewählt sind aus der Gruppe bestehend aus 7-[(1R,2R,3S,5S)-2-[[(5-hydroxybenzo[b]thien-3-yl)carbonyl]amino]-6,6-dimethylbicyclo[3.1.1]hept-3-yl]-, (5Z)-5-Heptensäure (S-5751), Laropiprant, und 2-[[4-[(1R,2S,3R,5R)-5-chloro-2-[(3S)-3-cyclohexyl-3-hydroxy-1-propyn-1-yl]-3-hydroxycyclopentyl]butyl]thio]-Essigsäure (TS-002), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Thromboxane Rezeptor Antagonist gelangen hierbei vorzugsweise Verbindungen zur Anwendung, die ausgewählt sind aus der Gruppe bestehend aus Seratrodast, N-[[(1,1-dimethylethyl)amino]carbonyl]-2-[(4-methylphenyl)amino]-5-nitro-Benzenesulfonamide (BM-573), (+/-)-sodium[2-(4-chlorophenylsulfonylaminomethyl)- indan-5-yl]acetate monohydrate (Z-335) und 2-[[[4-[[(4-chlorophenyl)sulfonyl]amino]butyl][[3-[[4-(1-methylethyl)-2-thiazolyl]methoxy]phenyl]methyl]amino]sulfonyl]-Benzoesäure (KP-496) gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Chemokine Rezeptor Antagonisten gelangen hierbei vorzugsweise Verbindungen zur Anwendung, die ausgewählt sind aus der Gruppe bestehend aus N-[5-chloro-2-[2-[(2R)-4-[(4-fluorophenyl)methyl]-2-methyl-1-piperazinyl]-2-oxoethoxy]phenyl]-Harnstoff hydrochloride (1:1) (BX-471), 2, N-[(1S,2S,4R)-4-(aminocarbonyl)-1-[(3-fluorophenyl)methyl]-2,7-dihydroxy-7-methyloctyl]- -Quinoxalinecarboxamide (CP-481715), (4,6-dimethyl-5-pyrimidinyl)[4-[(3S)-4-[(1R)-2-methoxy-1-[4-(trifluoromethyl)phenyl]ethyl]-3-methyl-1-piperazinyl]-4-methyl-1-piperidinyl]-Methanone (Sch-417690), 2-hydroxy-N,N-dimethyl-3-[[2-[[(1R)-1-(5-methyl-2-furanyl)propyl]amino]-3,4-dioxo-1-cyclobuten-1-yl]amino] - Benzamide (SCH-527123) und 1,4,8,11-Tetraazacyclotetradecane, 1,1'-[1,4-phenylenebis(methylene)]bis-, hydrochloride (1:8) (AMD-3100), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Neurokinin (NK1 oder NK2) Antagonisten gelangen hierbei vorzugsweise Verbindungen zur Anwendung, die ausgewählt sind aus der Gruppe bestehend aus: Saredutant, Nepadutant und Figopitant gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Sphingosinel-phosphat Rezeptor Modulatoren gelangen hierbei vorzugsweise Verbindungen zur Anwendung wie z.B. sonepcizumab, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Mucoregulatoren gelangen hierbei vorzugsweise Verbindungen zur Anwendung, die ausgewählt sind aus der Gruppe bestehend aus: 3-[2-oxo-2-[2-[[3-(trifluoromethyl)phenyl]amino]-3-pyridinyl]ethyl]-1(3H)-Isobenzofuranone (MSI-2216), Erdosteine, Fluorovent, Talniflumate, fudosteine, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als PPAR gamma Agonist gelangen hierbei vorzugsweise Verbindungen zur Anwendung, die ausgewählt sind aus der Gruppe bestehend aus: Rosiglitazone, Ciglitazone, Pioglitazone und N-[2-[2-[(3-fluorophenyl)imino]-4-[4-(4-morpholinyl)phenyl]-3(2H)-thiazolyl]ethyl]-N'-methyl-Harnstoff (SMP-028), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Rho Kinase Inhibitoren gelangen hierbei vorzugsweise Verbindungen zur Anwendung, wie z.B. Fasudil, gegebenenfalls in Form seiner Racemate, Enantiomere, Diastereomere und gegebenenfalls in Form seiner pharmakologisch verträglichen Säureadditionssalze, Prodrugs, Solvate oder Hydrate.

Als Adenosine Rezeptor Modulatoren gelangen hierbei vorzugsweise Verbindungen zur Anwendung, die ausgewählt sind aus der Gruppe bestehend aus 4-(3,4-dichlorophenyl)-5-(4-pyridinyl)-2-Thiazolamine (CGH-2466), 3-ethyl-3,9-dihydro-1-propyl-8-[1-[[3-(trifluoromethyl)phenyl]methyl]-1H-pyrazol-4-yl]-1H-Purine-2,6-dione (CVT-6883), N-(4-cyanophenyl)-2- [4-(2,3,6,9-tetrahydro-2,6-dioxo-1,3-dipropyl-1H-purin-8-yl)phenoxy] - Acetamide (MRS-1754), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Bradykinin Rezeptor Antagonisten gelangen hierbei vorzugsweise Verbindungen zur Anwendung, die ausgewählt sind aus der Gruppe bestehend aus Icatibant und 1-Piperazinepentanaminium, delta-amino-4-[[4-[[[2,4-dichloro-3-[[(2,4-dimethyl-8-chinolinyl)oxy]methyl]phenyl]sulfonyl]amino]tetrahydro-2H-pyran-4-yl]carbonyl]-N,N,N-trimethyl-ε-oxo-, chloride, hydrochloride (1:1:1), (deltaS)- (MEN-16132), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Endothelin Antagonisten gelangen hierbei vorzugsweise Verbindungen zur Anwendung, die ausgewählt sind aus der Gruppe bestehend aus Actelion-1, Ambrisentan, Sitaxsentan, N-(2-acetyl-4,6-dimethylphenyl)-3-[[(4-chloro-3-methyl-5-isoxazolyl)amino]sulfonyl]-2-Thiophenecarboxamid (TBC-3214) und Bosentan, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Interleukin 1-beta converting enzyme (ICE) Inhibitoren gelangen hierbei vorzugsweise Verbindungen zur Anwendung, die ausgewählt sind aus der Gruppe bestehend aus Pralnacasan und N-(4-amino-3-chlorobenzoyl)-3-methyl-L-valyl-N-[(2R,3 S)-2-ethoxytetrahydro-5-oxo-3-furanyl]-L-Prolinamid (=VX-765), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Toll-like Rezeptor (TLR) Modulatoren gelangen hierbei vorzugsweise Verbindungen zur Anwendung, die ausgewählt sind aus der Gruppe bestehend aus Resiquimod, Heplisav, Resatorvid (TAK-242), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als HMG-CoA Reductase Inhibitoren gelangen hierbei vorzugsweise Verbindungen zur Anwendung, die ausgewählt sind aus der Gruppe bestehend aus Cerivastatin, Pitavastatin, Rosuvastatin, Lovastatin, Simvastatin, Pravastatin, Fluvastatin und Avorvastatin, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als VLA-4 Antagonisten gelangen hierbei vorzugsweise Verbindungen zur Anwendung, die ausgewählt sind aus der Gruppe bestehend aus Natalizumab, valategrast, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als SHIP Agonisten gelangen hierbei vorzugsweise Verbindungen zur Anwendung, die ausgewählt sind aus der Gruppe bestehend aus 2,3,4,4a,5,6,6a, 11,11a,11b-decahydro-4,4,6a,7,11b-pentamethyl-, (4aS,6aR,11aR,11bS)-1H-Benzo[a]fluoren-9-ol (AQX-MN100) und MN-106, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als anti-TNF-Antikörper gelangen hierbei vorzugsweise Verbindungen zur Anwendung, die ausgewählt sind aus der Gruppe bestehend aus Infliximab, Adalimumab, Golimumab. CytoFab und Etanercept.

Als Substanzen gegen Schwellungen der Atemwege gelangen hierbei vorzugsweise Verbindungen zur Anwendung, die ausgewählt sind aus der Gruppe bestehend aus Phenylephrine, Phenylpropanolamine, Pseudophedrine, Oxymetazoline, Epinephrine, Naphazoline, Xylometazoline, Propylhexedrine und Llevo-desoxyephedrine, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen gegen Husten gelangen hierbei vorzugsweise Verbindungen zur Anwendung, die ausgewählt sind aus der Gruppe bestehend aus Hydrocodone, Caramiphen, Carbetapentane und Dextramethorphan, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter Lipoxin A4 Derivative gelangen hierbei vorzugsweise Verbindungen zur Anwendung, die ausgewählt sind aus der Gruppe bestehend aus
- 7,9,11,13-Eicosatetraensäure, 5,6,15-trihydroxy-, (5S,6R,7E,9E,11Z,13E,15R)-(15-epilipoxin a4),
- 7,9,11,13-Eicosatetraensäure, 16-(4-fluorophenoxy)-5,6,15-trihydroxy-, (5S,6R,7E,9E,11Z,13E,15S)-(ATL-1),
- Aspirin-getriggeres Lipoxin A(4) and Analoge,
- protectin D1 (4,7,11,13,15,19-Docosahexaensäure, 10,17-dihydroxy-, (4Z,7Z,10R,11E,13E,15Z,17S,19Z)-,
- Resolvin E1 (6,8,10,14,16-Eicosapentaensäure, 5,12,18-trihydroxy-, (5S,6Z,8E,10E,12R,14Z,16E,18R)-) and
- Benzo-Lipoxin A4 Analoge,
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter FPRL1-Modulatoren gelangen hierbei vorzugsweise Verbindungen zur Anwendung, wie z.B. 5(S),6(R), 7-trihydroxyheptanonsäure-Methyl-ester, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter PI3 Kinase Antagonisten gelangen hierbei vorzugsweise Verbindungen zur Anwendung, die ausgewählt sind aus der Gruppe bestehend aus
- 5-(Quinoxalin-6-ylmethylene)thiazolidine-2,4-dione (AS-605240),
- 2-[(6-amino-9H-purin-9-yl)methyl]-5-methyl-3-(2-methylphenyl)-4(3H)-Quinazolinone (C-87114),
- 2-Methyl-2-[4-[3-methyl-2-oxo-8-(chinolin-3-yl)-2,3-dihydroimidazo[4,5-c]chinolin-1-yl]phenyl]propionitrile (BEZ-235),
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter CCR5 Antagonisten gelangen hierbei vorzugsweise Verbindungen zur Anwendung, die ausgewählt sind aus der Gruppe bestehend aus
- Maraviroc (4,4-difluoro-N-[(1S)-3-[(3-exo)-3-[3-methyl-5-(1-methylethyl)-4H-1,2,4-triazol-4-yl]-8-azabicyclo[3.2.1]oct-8-yl]-1-phenylpropyl]-Cyclohexancarboxamid), CCR5mAb004
- Vicriviroc ((4,6-dimethyl-5-pyrimidinyl)[4-[(3S)-4-[(1R)-2-methoxy-1-[4-(trifluoromethyl)phenyl]ethyl]-3-methyl-1-piperazinyl]-4-methyl-1-piperidinyl] - Methanon) und
- Nifeviroc (N-[1-[[(3S, 4R)-1-(cyclopentylcarbonyl)-4-hydroxy-4-phenyl-3-pyrrolidinyl]methyl]-4-piperidinyl]-N-2-propen-1-yl-(4-nitrophenyl)methyl ester-Carbaminsäure),
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter CXCR1 or CXCR2 Antagonisten gelangen hierbei vorzugsweise Verbindungen zur Anwendung, wie z.B. 3-[[3-[(Dimethylamino)carbonyl]-2-hydroxyphenyl]amino]-4-[[(R)-1-(5-methylfuran-2-yl)propyl]amino]cyclobut-3-ene-1,2-dione (SCH-527123),
gegebenenfalls in Form seiner Racemate, Enantiomere, Diastereomere und gegebenenfalls in Form seiner pharmakologisch verträglichen Säureadditionssalze, Prodrugs, Solvate oder Hydrate.

Erfindungsgemäß bevorzugt sind die Säureadditionssalze der oben genannten MAP Kinase Inhibitoren, iNOS-Inhibitoren, MRP4-Inhibitoren, Leukotriene Biosynthese Inhibitoren, Nicht-steroidalen antientzündlichen Agenzien (NSAIDs), CRTH2 Antagonisten, DP1-Rezeptor Modulatoren, Thromboxane Rezeptor Antagonisten, Chemokine Rezeptor Antagonisten, Neurokinin (NK1 oder NK2) Antagonisten, Sphingosinel-phosphat Rezeptor Modulatoren, Mucoregulatoren, PPAR gamma Agonisten, Rho Kinase Inhibitoren, Adenosine Rezeptor Modulatoren, Bradykinin Rezeptor Antagonisten, Endothelin Antagonisten, Interleukin 1-beta converting enzyme (ICE) Inhibitoren, Toll-like Rezeptoren (TLR) Modulatoren, HMG-CoA Reductase Inhibitoren, VLA-4 Antagonisten, SHIP Agonisten, anti-TNF-Antikörper, Substanzen gegen Schwellungen der Atemwege, Substanzen gegen Husten, Lipoxin A4 Derivative, PI3 Kinase Antagonisten, FPRL1-Modulatoren, CCR5 Antagonisten , CXCR1- oder CXCR2-Antagonisten ebenfalls ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

### Bezugszeichenliste

- 1: Kapselkappe
- 2: Kapselkörper
- 3: Sicke (an Kapselkappe)
- 4: Sicke (an Kapselkappe)
- 5: Wulst (an Kapselkörper)
- 6: Loch (an Kapselkappe)
- 7: Loch (an Kapselkörper)
- 8: Steg (an Kapselkörper)
- 9: Nut (an Kapselkappe)
- 11: Kapsel in erster Einsteckstellung (Löcher verdeckt)
- 12: Kapsel in zweiter Einsteckstellung (Löcher geöffnet)
- 13: Kapselkammer
- 13a: Längswand (der Kapselkammer)
- 14: Schieber
- 14a: eingeschobene Längswand (am Schieber)
- 14b: Vorsprung (am Schieber)
- 15: Sieb
- 16: Schacht
- 17: Kanal
- 18: Führungsschiene
- 19: Feder
- 20: Lufteinlassöffnung
- 21: Anschlagkante
- 22: Ring
- 23: Aufnahme für Ring
- 23a: Rastelement (an Aufnahme für Ring)

## Patentansprüche

1. Kapsel zur Verwendung in einem kapselbasierten Inhalator,
wobei die Kapsel eine pulverförmige pharmazeutische Zubereitung zur Inhalation enthält, und zwei einseitig offene Kapselelemente, nämlich einen Kapselkörper (2) und eine Kapselkappe (1), umfasst, die teleskopartig über ihre Öffnungen ineinander gesteckt werden können, so dass ein Hohlraum gebildet wird,
und die zusammengefügte Kapsel eine Länge von 9 bis 22 Millimeter und einen äußeren Durchmesser von 4 bis 10 Millimeter und einen Kapselmantel aufweist,
**dadurch gekennzeichnet, dass** Kapselkörper (2) und Kapselkappe (1) beide einen Kapselmantelbereich und zusätzlich zur einseitigen Öffnung je zwei vorgefertigte Löcher (7a, 7b, 6a, 6b) im Kapselmantelbereich aufweisen , und
dass die Kapsel hinsichtlich des teleskopartigen Ineinandersteckens von Kapselkörper (2) und Kapselkappe (1) zwei Einsteckstellungen aufweist, in denen Kapselkörper (2) und Kapselkappe (1) verrasten, wobei in einer ersten Einsteckstellung Kapselkörper (2) und Kapselkappe (1) so ineinander gesteckt sind, dass die vorgefertigten Löcher (7a, 7b, 6a, 6b) in Kapselkörper (2) und Kapselkappe (1) verdeckt sind und somit der Hohlraum in der Kapsel (11) geschlossen ist, und in einer zweiten Einsteckstellung Kapselkörper (2) und Kapselkappe (1) so ineinander gesteckt sind, dass die vorgefertigten Löcher (7a, 7b, 6a, 6b) in Kapselkörper (2) und Kapselkappe (1) sich so überdecken, so dass in der zweiten Einsteckstellung die Kapsel (12) im Kapselmantelbereich zwei Löcher, jeweils eins in der Nähe eines Kapselendes, aufweist.

2. Kapsel nach Anspruch 1, **dadurch gekennzeichnet, dass** Kapselkörper (2) und Kapselkappe (1) becherartig ausgebildet sind und der jeweilige Kapselmantelbereich eine zylindrisch oder elliptisch umlaufende Wand bildet.

3. Kapsel nach Anspruch 2, **dadurch gekennzeichnet, dass** die vorgefertigten Löcher (7, 6) in Kapselkörper (2) und Kapselkappe (1) im umlaufenden Wandbereich des jeweiligen Kapselmantelbereichs angeordnet sind.

4. Kapsel nach Anspruch 3, **dadurch gekennzeichnet, dass** die Kapselmantelbereiche von Kapselkörper (2) und Kapselkappe (1) auf den Seiten, die im eingesteckten Zustand aneinander liegen, eine Strukturierung aufweisen, die das Ineinanderstecken der beiden Kapselelemente nur in definierter Ausrichtung zueinander zulässt.

5. Kapsel nach einem der vorherigen Ansprüche, **dadurch gekennzeichnet, dass** es hinsichtlich des teleskopartigen Ineinandersteckens von Kapselkörper (2) und Kapselkappe (1) eine Einsteckstellung gibt, in der vorgefertigte Löcher (7a, 7b, 6a, 6b) in Kapselkörper (2) und Kapselkappe (1) sich jeweils so überdecken, dass die ansonsten geschlossene Kapsel zwei Löcher an den entsprechenden Stellen aufweist.

6. Kapsel nach einem der vorherigen Ansprüche, **dadurch gekennzeichnet, dass** Kapselkörper (2) und Kapselkappe (1) aus der ersten Einsteckstellung durch ein Zusammenschieben von Kapselkörper (2) und Kapselkappe (1) in die zweite Einsteckstellung, in der die Löcher zum Hohlraum der Kapsel (11) hin freigelegt sind, gelangen.

7. Kapsel nach einem der vorherigen Ansprüche **dadurch gekennzeichnet, dass** sich Kapselkörper (2) und Kapselkappe (1) mit einander wechselwirkende Rastelemente aufweisen, die bewirken, dass sich die beiden Kapselelemente nach dem Ineinanderstecken bis zur ersten Einsteckstellung nicht mehr zerstörungsfrei voneinander trennen lassen und/oder dass die relative Position der beiden Kapselelemente zueinander in einer Einsteckstellung fixiert ist.

8. Kapsel nach einem der Ansprüche 5 bis 7 **dadurch gekennzeichnet, dass** die vorgefertigten Löcher (7a, 7b, 6a, 6b) in Kapselkörper (2) und Kapselkappe (1) durch entsprechendes Ineinanderstecken von Kapselkörper (2) und Kapselkappe (1) in Überdeckung gebracht werden können, und dass von diesen Löchern (7a, 7b, 6a, 6b) eines - insbesondere das Loch (7) im äußeren Kapselelements - größer als das jeweils andere, vorzugsweise als Langloch oder elliptisch, ausgebildet ist.

9. Kapsel nach einem der vorherigen Ansprüche, **dadurch gekennzeichnet, dass** die Kapselelemente aus einem Kunststoffmaterial, vorzugsweise beide aus dem gleichen Material und insbesondere aus Polyethylen, Polycarbonat, Polyester, Polypropylen oder Polyethylenterrephthalat, in einem Spritzgussverfahren gefertigt sind und die vorgefertigten Löcher (7a, 7b, 6a, 6b) bereits im gleichen Spritzgussverfahren ausgebildet worden sind.

10. Inhalator mit einer Kapselkammer (13) in die eine Kapsel gemäß einer der vorherigen Ansprüche aufgenommen ist, wobei
- die Kapselkammer (13) einen Lufteinlass und einen in Richtung eines Mundstücks führenden Luftauslass aufweist,
- die Kapsel eine pharmazeutische, vorzugsweise pulverförmige Zubereitung enthält und
- die Kapsel in die Kapselkammer (13) eingesetzt ist oder aus einem Vorratsmagazin in die Kapselkammer (13) eingeführt werden kann,
**dadurch gekennzeichnet, dass** der Inhalator einen Schieber (14) aufweist, bei dessen Betätigung Teile der Kapsel (11) - insbesondere Kapselkappe (1) zu Kapselkörper (2) - relativ so zueinander verschoben werden, dass an der Kapsel die zwei Löcher freigegeben werden.

11. System aus einem Inhalator und mindestens einer Kapsel (11) nach einem der vorherigen Ansprüche, **dadurch gekennzeichnet, dass**
- der Inhalator eine Kapselkammer (13) mit einem Lufteinlass und einem in Richtung eines Mundstücks führenden Luftauslass aufweist,
- vor Betätigung des Inhalators die Kapsel (11) hinsichtlich des teleskopartigen Ineinandersteckens von Kapselkörper (2) und Kapselkappe (1) in ihrer erste Einsteckstellung vorliegt,
- die Kapsel (11) in die Kapselkammer eingesetzt ist oder aus einem Vorratsmagazin in die Kapselkammer (13) eingeführt werden kann und
- der Inhalator einen Schieber (14) aufweist, mit dem die Kapsel (11) in der Kapselkammer (13) derart zusammengeschoben werden kann, dass die zweite Einsteckstellung erreicht wird.

12. System aus Inhalator und Kapsel (11) nach Anspruch 11 **dadurch gekennzeichnet, dass** der Schieber (14) mit einer Feder (19) derart zusammenwirkt, dass er nach Betätigung wieder in seine Ausgangslage zurückgestellt wird.

## Claims

1. Capsule for use in an capsule-based inhaler, the capsule comprising a powdered pharmaceutical preparation for inhalation and two capsule elements open at one end, namely a capsule body (2) and a capsule cap (1), which can be fitted into one another telescopically through their openings, so as to form a cavity,
and the assembled capsule having a length of 9 millimeter to 22 millimeter and an external diameter of 4 millimeter to 10 millimeter and a capsule casing,
**characterised in that** the capsule body (2) and capsule cap (1) both comprise a capsule casing region and in addition to the opening at one end two prefabricated holes (7a, 7b , 6a, 6b) in the capsule casing region in each case, and
that the capsule comprises two insertion positions with regards to capsule body (2) and capsule cap (1) being fitted telescopically into one another, the capsule body (2) and the capsule cap (1) latching together in the two positions, wherein in a first insertion position capsule body (2) and capsule cap (1) are fitted into one another such that the prefabricated holes (7a, 7b , 6a, 6b) in capsule body (2) and capsule cap (1) are covered and the cavity of the capsule (11) is closed off, and in a second insertion position capsule body (2) and capsule cap (1) are fitted into one another such that the prefabricated holes (7a, 7b , 6a, 6b) in the capsule body (2) and capsule cap (1) overlap with one another, such that in the second insertion position the capsule (12) comprises two holes in the capsule casing region, one hole close to a capsule end in each case.

2. Capsule according to claim 1, **characterised in that** the capsule body (2) and capsule cap (1) are of cup-shaped configuration and the respective capsule casing region forms a cylindrically or elliptically encircling wall.

3. Capsule according to claim 2, **characterised in that** the prefabricated holes (7, 6) in the capsule body (2) and capsule cap (1) are arranged in the encircling wall region of the respective capsule casing region.

4. Capsule according to claim 3, **characterised in that** the capsule casing regions of the capsule body (2) and capsule cap (1) comprise, on the sides that abut on one another in the inserted state, a structuring that enables the two capsule elements to be fitted into one another only in a defined alignment.

5. Capsule according to one of the preceding claims, **characterised in that** with respect to the telescopic fitting together of the capsule body (2) and capsule cap (1), there is an insertion position in which prefabricated holes (7a, 7b , 6a, 6b) in the capsule body (2) and capsule cap (1) are in registry with one another such that the otherwise closed capsule comprises two holes at the corresponding locations.

6. Capsule according to one of the preceding claims, **characterised in that** capsule body (2) and capsule cap (1) get from the first insertion position into the second insertion position by the capsule body (2) and capsule cap (1) being pushed together, wherein the holes into the cavity of the capsule (11) are exposed in the second insertion position.

7. Capsule according to one of the preceding claims **characterised in that** the capsule body (2) and capsule cap (1) comprise latching elements interacting with one another which have the effect that the two capsule elements after being fitted into one another up to the first insertion position can no longer be separated from one another non-destructively and/or that the relative position of the two capsule elements relative to one another is fixed in an insertion position.

8. Capsule according to one of claims 5 to 7, **characterised in that** prefabricated holes (7a, 7b , 6a, 6b) in capsule body (2) and capsule cap (1) can be brought into registry by fitting capsule body (2) and capsule cap (1) into one another accordingly, and **in that**, of these holes (7a, 7b , 6a, 6b), one, particularly the hole (7) in the outer capsule element, is larger than the respective other one which is preferably embodied as an oblong hole or an elliptical hole.

9. Capsule according to one of the preceding claims, **characterised in that** the capsule elements are made from a plastics material, preferably both from the same material and, in particular, from polyethylene, polycarbonate, polyester, polypropylene or polyethylene terephthalate, in an injection moulding process and the prefabricated holes (7a, 7b , 6a, 6b) have already been formed in the same injection moulding process.

10. Inhaler having a capsule chamber (13) in which a capsule according to one of the preceding claims is accommodated, wherein
the capsule chamber (13) comprises an air inlet and an air outlet leading towards a mouthpiece,
the capsule contains a preferably powdered pharmaceutical preparation and
the capsule is inserted in the capsule chamber (13) or can be inserted in the capsule chamber (13) from a storage magazine,
**characterised in that**
the inhaler comprises a pusher (14), which when actuated causes parts of the capsule (11) to be moved relative to each other, particularly capsule cap (1) to be moved relative to capsule body (2), such that the two holes are exposed on the capsule.

11. System comprising an inhaler and at least one capsule (11) according to one of the preceding claims, **characterised in that**
- the inhaler comprises a capsule chamber (13) with an air inlet and an air outlet leading in the direction of a mouthpiece,
- before actuation of the inhaler the capsule (11) is in its first insertion position with respect to the telescopic fitting together of the capsule body (2) and capsule cap (1),
- the capsule (11) is inserted in the capsule chamber (13) or can be inserted in the capsule chamber (13) from a storage magazine and
- the inhaler comprises a pusher (14) with which the capsule (11) in the capsule chamber (13) can be pushed together such that the second insertion position is reached.

12. System comprising an inhaler and capsule (11) according to claim 11, **characterised in that** the pusher (14) cooperates with a spring (19) such that after actuation it is restored to its original position.

## Revendications

1. Capsule à utiliser dans un inhalateur basé sur des capsules,
dans laquelle la capsule contient une préparation pharmaceutique pulvérulente pour l'inhalation, et deux éléments de capsule ouverts d'un côté, à savoir un corps de capsule (2) et un capuchon de capsule (1), qui peuvent être enfichés l'un dans l'autre de manière télescopique via leurs ouvertures, de sorte qu'une cavité est formée,
et la capsule assemblée présente une longueur de 9 à 22 millimètres et un diamètre extérieur de 4 à 10 millimètres et une enveloppe de capsule,
**caractérisée en ce que** le corps de capsule (2) et le capuchon de capsule (1) présentent tous deux une zone d'enveloppe de capsule et en plus de l'ouverture unilatérale respectivement deux trous préfabriqués (7a, 7b, 6a, 6b) dans la zone d'enveloppe de capsule, et
**que** la capsule en ce qui concerne l'emboîtement télescopique du corps de capsule (2) et du capuchon de capsule (1) présente deux positions d'enfichage, dans lesquelles le corps de capsule (2) et le capuchon de capsule (1) s'enclenchent, dans laquelle dans une première position d'enfichage le corps de capsule (2) et le capuchon de capsule (1) sont enfichés l'un dans l'autre de sorte que les trous préfabriqués (7a, 7b, 6a, 6b) dans le corps de capsule (2) et le capuchon de capsule (1) sont cachés et ainsi la cavité dans la capsule (11) est fermée, et dans une deuxième position d'enfichage le corps de capsule (2) et le capuchon de capsule (1) sont enfichés l'un dans l'autre de sorte que les trous préfabriqués (7a, 7b, 6a, 6b) dans le corps de capsule (2) et le capuchon de capsule (1) se recouvrent de sorte que dans la deuxième position d'enfichage la capsule (12) présente dans la zone d'enveloppe de capsule deux trous, respectivement un à proximité d'une extrémité de capsule.

2. Capsule selon la revendication 1, **caractérisée en ce que** le corps de capsule (2) et le capuchon de capsule (1) sont réalisés à la manière d'un gobelet et la zone d'enveloppe de capsule respective forme une paroi circonférentielle cylindrique ou elliptique.

3. Capsule selon la revendication 2, **caractérisée en ce que** les trous préfabriqués (7, 6) sont agencés dans le corps de capsule (2) et le capuchon de capsule (1) dans la zone de paroi circonférentielle de la zone d'enveloppe de capsule respective.

4. Capsule selon la revendication 3, **caractérisée en ce que** les zones d'enveloppe de capsule du corps de capsule (2) et du capuchon de capsule (1) présentent sur les côtés, qui se trouvent l'un contre l'autre à l'état enfiché, une structuration, qui permet l'emboîtement des deux éléments de capsule seulement dans une orientation définie.

5. Capsule selon l'une quelconque des revendications précédentes, **caractérisée en ce qu'**il y a en ce qui concerne l'emboîtement télescopique du corps de capsule (2) et du capuchon de capsule (1) une position d'enfichage, dans laquelle des trous préfabriqués (7a, 7b, 6a, 6b) dans le corps de capsule (2) et le capuchon de capsule (1) se recouvrent respectivement de sorte que la capsule sinon fermée présente deux trous aux endroits correspondants.

6. Capsule selon l'une quelconque des revendications précédentes, **caractérisée en ce que** le corps de capsule (2) et le capuchon de capsule (1) parviennent de la première position d'enfichage par un rapprochement du corps de capsule (2) et du capuchon de capsule (1) dans la deuxième position d'enfichage, dans laquelle les trous de la cavité de la capsule (11) sont dégagés.

7. Capsule selon l'une quelconque des revendications précédentes, **caractérisée en ce que** le corps de capsule (2) et le capuchon de capsule (1) présentent des éléments d'encliquetage interagissant l'un avec l'autre, qui entraînent le fait que les deux éléments de capsule après l'emboîtement jusqu'à la première position d'enfichage ne peuvent plus être séparés l'un de l'autre sans destruction et/ou que la position relative des deux éléments de capsule l'un par rapport à l'autre est fixée dans une position d'enfichage.

8. Capsule selon l'une quelconque des revendications 5 à 7, **caractérisée en ce que** les trous préfabriqués (7a, 7b, 6a, 6b) dans le corps de capsule (2) et le capuchon de capsule (1) peuvent être mis en coïncidence par emboîtement correspondant du corps de capsule (2) et du capuchon de capsule (1), et que de ces trous (7a, 7b, 6a, 6b) un - en particulier le trou (7) dans l'élément de capsule extérieur - est réalisé plus grand que respectivement l'autre, de préférence en tant que trou oblong ou de manière elliptique.

9. Capsule selon l'une quelconque des revendications précédentes, **caractérisée en ce que** les éléments de capsule sont fabriqués en un matériau plastique, de préférence les deux dans le même matériau et en particulier en polyéthylène, polycarbonate, polyester, polypropylène ou polytéréphtalate d'éthylène, selon un procédé de moulage par injection et les trous préfabriqués (7a, 7b, 6a, 6b) sont déjà réalisés selon le même procédé de moulage par injection.

10. Inhalateur avec une chambre de capsule (13) dans laquelle est reçue une capsule selon l'une quelconque des revendications précédentes, dans lequel
- la chambre de capsule (13) présente une entrée d'air et une sortie d'air conduisant en direction d'un embout,
- la capsule contient une préparation pharmaceutique, de préférence pulvérulente et
- la capsule est insérée dans la chambre de capsule (13) ou peut être introduite d'un magasin de stockage dans la chambre de capsule (13),
**caractérisé en ce que** l'inhalateur présente un coulisseau (14), lors de l'actionnement duquel des parties de la capsule (11) - en particulier le capuchon de capsule (1) par rapport au corps de capsule (2) - sont décalées l'une par rapport à l'autre de sorte que les deux trous sont dégagés au niveau de la capsule.

11. Système formé d'un inhalateur et d'au moins une capsule (11) selon l'une quelconque des revendications précédentes, **caractérisé en ce que**
- l'inhalateur présente une chambre de capsule (13) avec une entrée d'air et une sortie d'air conduisant en direction d'un embout,
- avant l'actionnement de l'inhalateur, la capsule (11) est présente en ce qui concerne l'emboîtement télescopique du corps de capsule (2) et du capuchon de capsule (1) dans sa première position d'enfichage,
- la capsule (11) est insérée dans la chambre de capsule ou peut être introduite d'un magasin de stockage dans la chambre de capsule (13) et
- l'inhalateur présente un coulisseau (14), avec lequel la capsule (11) peut être rapprochée dans la chambre de capsule (13) de sorte que la deuxième position d'enfichage est atteinte.

12. Système formé de l'inhalateur et de la capsule (11) selon la revendication 11, **caractérisé en ce que** le coulisseau (14) coopère avec un ressort (19) de sorte qu'il est ramené dans sa position de départ après l'actionnement.
